# EUROPEAN PATENT APPLICATION

(11) **EP 3 584 236 A1**
(43) Date of publication of application: **25.12.2019**
(21) Application number: 18382445.7
(22) Date of filing: 20.06.2018
(51) Int. Cl.: C07C 271/56, C07C 275/26, C07C 335/16, C07D 211/34, C07D 211/58, C07D 211/96, C07D 277/82, C07D 451/04, C07D 493/08, A61K 31/27, A61K 31/17, A61P 9/00, A61P 29/00, A61P 25/00

(54) **POLYCYCLIC COMPOUNDS SOLUBLE EPOXIDE HYDROLASE INHIBITORS**

(71) Applicant: Universitat de Barcelona, 08028 Barcelona (ES)
(72) Inventor: CODONY I GISBERT, Sandra, E-08006 Barcelona (ES); GALDEANO CANTADOR, Carlos, E-08328 Alella, Barcelona (ES); LEIVA MARTÍNEZ, Rosana, E-08028 Barcelona (ES); LARISA TURCU, Andreea, E-08812 Sant Pere de Ribes, Barcelona (ES); VALVERDE MURILLO, Elena, E-43711 Banyeres del Penedès, Tarragona (ES); VÁZQUEZ CRUZ, Santiago, E-08940 Cornellá del Llobregat, Barcelona (ES)
(74) Representative: ABG Intellectual Property Law, S.L.

(57) **Abstract**

The present invention relates to soluble epoxide hydrolase (sEH) inhibitors of formula (I) 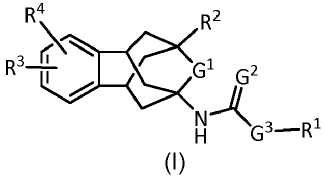 to processes for their obtention and to their therapeutic indications.

## Description

The present invention relates to the field of pharmaceutical products for human and veterinary medicine, particularly to soluble epoxide hydrolase (sEH) inhibitors and their therapeutic indications.

### BACKGROUND ART

A total of more than 100 patent publications have described multiple classes of sEH inhibitors, based on different chemical structures, such as amides, thioamides, ureas, thioureas, carbamates, acyl hydrazones and chalcone oxides (cf. e.g. H.C. Shen, "Soluble epoxide hydrolase inhibitors: a patent review", Expert Opin Ther Patents 2010, vol. 20, pp. 941-956, a review with 149 references).

sEH inhibition has been associated to various beneficial biological effects, that may be translated into various therapeutic treatments (cf. e.g. H.C. Shen and B.D. Hammock, "Discovery of inhibitors of soluble epoxide hydrolase: A target with multiple potential therapeutic indications", J Med Chem. 2012, vol. 55, pp. 1789-1808, a review with 117 references; K.M. Wagner et al. "Soluble epoxide hydrolase as a therapeutic target for pain, inflammatory and neurodegenerative diseases", Pharmacol Ther. 2017, vol 180, pp 62-76, a review with 186 references).

More specifically the documents cited below have described the usefulness of sEH inhibition in the treatment of the following diseases: Hypertension (Recent Pat Cardiovasc Drug Discov. 2006 Jan;1(1):67-72), atherosclerosis (J Cardiovasc Pharmacol. 2008 Oct;52(4):314-23), pulmonary diseases (Am J Respir Cell Mol Biol. 2012 May;46(5):614-22 / Am J Respir Crit Care Med. 2014 Oct 15;190(8):848-50), kidney diseases (Bioorg Med Chem Lett. 2014 Jan 15;24(2):565-70 / Am J Physiol Renal Physiol. 2013 Jan 15;304(2):F168-76 / Am J Physiol Renal Physiol. 2014 Oct 15;307(8):F971-80), stroke (J Biol Chem. 2014 Dec 26;289(52):35826-38 / PLoS One. 2014 May 13;9(5):e97529), pain (J Agric Food Chem. 2011 Apr 13;59(7):2816-24 / Inflamm Allergy Drug Targets. 2012 Apr;11 (2):143-58), neuropathic pain (J Agric Food Chem. 2011 Apr 13;59(7):2816-24 / Drug Discov Today 2015 Nov;20(11):1382-90 / Proc Natl Acad Sci U S A. 2015 Jul 21;112(29):9082-7), inflammation (Inflamm Allergy Drug Targets. 2012 Apr;11(2):143-58 / Proc Natl Acad Sci U S A. 2005 Jul 12;102(28):9772-7), pancreatitis in particular acute pancreatitis (Mol Pharmacol. 2015 Aug;88(2):281-90), immunological disorders (WO 00/23060 A2), eye diseases (WO 2007/009001 A1) in particular diabetic keratopathy (Diabetes. 2018 Jun;67(6):1162-1172), wet age-related macular degeneration (ACS Chem Biol. 2018 Jan 19; 13:45-52) and retinopathy (Nature. 2017 Dec 14;552(7684):248-252) such as premature retinopathy and diabetic retinopathy, cancer (Prog Lipid Res. 2014 Jan;53:108-23), obesity (Nutr Metab Cardiovasc Dis. 2012 Jul;22(7):598-604), including obesity-induced colonic inflammation (Proc Natl Acad Sci U S A. 2018 May 15;115(20):5283-5288), diabetes (Proc Natl Acad Sci U S A. 2011 May 31;108(22):9038-43), metabolic syndrome (Exp Diabetes Res. 2012;2012:758614), preeclampsia (Med. Hypotheses, 2017 Oct; 108:81-5), anorexia nervosa ("Pharmacokinetic optimization of six soluble epoxide hydrolase inhibitors for the therapeutic use in a murine model of anorexia" Abstracts of Papers, 241st ACS National Meeting & Exposition, Anaheim, CA, United States, March 27-31, 2011 (2011), MEDI-92), depression (J Neurosci Res. 2017 Dec;95(12):2483-2492), erectile dysfunction (Phytother Res. 2016 Jul;30(7):1119-27), wound healing (J Surg Res. 2013 Jun 15;182(2):362-7), NSAID-induced ulcers (J Pharmacol Exp Ther. 2016 Jun;357(3):529-36), emphysema (Am J Respir Cell Mol Biol. 2012 May;46(5):614-22), scrapie (Life Sci. 2013 Jun 21;92(23):1145-50), Parkinson's disease (Mol Neurobiol. 2015 Aug;52(1):187-95), arthritis (Drug Metab Dispos. 2015 May;43(5):788-802), arrhythmia (Cardiovasc Ther. 2011 Apr;29(2):99-111), Alzheimer's disease (Pharmacol Ther. 2017 Dec; 180:62-76), Raynaud's syndrome (WO 2003/002555 A1), cardiomyopathy (Int J Cardiol. 2012 Mar 8;155(2):181-7), vascular cognitive impairment (Prostaglandins Other Lipid Mediat. 2014 Oct;113-115:30-7), mild cognitive impairment (Pharmacol Ther. 2017 Dec;180:62-76), inflammatory bowel diseases (Dig Dis Sci. 2012 Oct;57(10):2580-91), cirrhosis (Toxicol Appl Pharmacol. 2015 Jul 15;286(2): 102-11), osteoporosis (FASEB J. 2015 Mar;29(3):1092-101), chronic periodontitis (J Pharmacol Exp Ther. 2017 Jun;361(3):408-416), sepsis (FASEB J. March 2008 22 (Meeting Abstract Supplement) 479.17), seizure disorders (epilepsy) (PLoS One. 2013 Dec 11;8(12):e80922), dementia (Prostaglandins Other Lipid Mediat. 2014 Oct; 113-115:30-7), edema (Stroke. 2015 Jul;46(7):1916-22.), attention-deficit hyperactivity disorder (WO 2017/120012 A1), schizophrenia (Proc Natl Acad Sci U S A. 2016 Mar 29;113(13):E1944-52), drug dependency (WO 2017/120012 A1), social anxiety (WO 2017/120012 A1), colitis (Anticancer Res. 2013 Dec;33(12):5261-5271), amyotrophic lateral sclerosis (WO 2016/133788 A1), chemotherapy induced side effects (Toxicology. 2017 Aug 15;389:31-41), laminitis (Equine Vet J. 2017 May;49(3):345-351), inflammatory joint pain and synovitis (J Vet Pharmacol Ther. 2018 Apr;41(2):230-238), endothelial dysfunction (Prostaglandins Other Lipid Mediat. 2017 Jul;131:67-74), subarachnoid hemorrhage (Stroke. 2015 Jul;46(7):1916-22), including aneurysmal subarachnoid hemorrhage (J Neurosurg Anesthesiol. 2015 Jul; 27(3):222-240), traumatic brain injury (Oncotarget. 2017 Sep 21;8(61):103236-60) and diabetes-induced learning and memory impairment (Prostaglandins Other Lipid Mediat. 2018 May;136:84-89).

Despite the high inhibitory activity of many of the reported sEH inhibitory compounds, until now no sEH inhibitor has reached the market. Thus, there is a need to develop new sEH inhibitors.

The inventors have now found a new family of polycyclic compounds having high inhibitory activity for soluble epoxide hydrolase.

### SUMMARY OF INVENTION

An aspect of the present invention relates to the provision of compounds of formula (I) 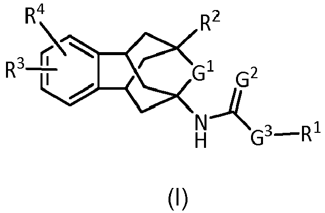 or a stereoisomer or a pharmaceutically acceptable salt thereof, wherein:
G¹ represents an oxygen atom or a methylene group or a single bond;
G² represents an oxygen atom or a sulphur atom;
G³ represents a radical selected from the group consisting of -NH-(CH₂)ₘ₋, -O-(CH₂)ₘ- and -(CH₂)ₙ-;
m is an integer from 0 to 6;
n is an integer from 1 to 7;
R¹ is a radical selected from the group consisting of:
   a)C₆-C₁₀ aryl which may be optionally substituted by 1 to 4 substituents selected from the group consisting of halogen atoms, C₁-C₆ acyl, nitro (NO₂), cyano (C≡N), trifluoromethyl (CF₃), trifluoromethoxy (OCF₃), pentafluorosulfanyl (SF₅), sulfonyl (SO₃H), fluorosulfonyl (SO₂F), carboxylic group (COOH), amino (NH₂), mono-C₁-C₆ alkylamino, di-C₁-C₆ alkylamino, C₁-C₆ alkoxy, C₁-C₆ alkyl, C₁-C₆ alkoxycarbonylmethyl and methylaminocarbonylpyridyloxy;
   b)heteroaryl having from 2 to 11 carbon atoms and 1, 2 or 3 heteroatoms selected from the group consisting of N, O and S and which may be optionally substituted by 1 to 4 substituents selected from the group consisting of halogen atoms, C₁-C₆ acyl, nitro (NO₂), cyano (C≡N), trifluoromethyl (CF₃), trifluoromethoxy (OCF₃), pentafluorosulfanyl (SF₅), sulfonyl (SO₃H), fluorosulfonyl (SO₂F), carboxylic group (COOH), amino (NH₂), mono-C₁-C₆ alkylamino, di-C₁-C₆ alkylamino, C₁-C₆ alkoxy, C₁-C₆ alkyl and C₁-C₆ alkoxycarbonylmethyl;
   c)saturated or partially unsaturated, monocyclic or bicyclic heterocyclyl having from 5 to 11 carbon atoms and 1, 2 or 3 heteroatoms selected from the group consisting of N, O and S and which may be optionally substituted by 1 to 4 substituents selected from the group consisting of halogen atoms, C₁-C₆ acyl, C₃-C₆ cycloalkyl-C(=O), nitro (NO₂), cyano (C≡N), trifluoromethyl (CF₃), trifluoromethylcarbonyl (CF₃CO), pentafluorosulfanyl (SF₅), sulfonyl (SO₃H), carboxylic group (COOH), amino (NH₂), mono-C₁-C₆ alkylamino, di-C₁-C₆ alkylamino, C₁-C₆ alkoxy, C₁-C₆ alkyl, C₁-C₆ alkoxycarbonylmethyl, C₁-C₆ alkylsulfonyl, C₃-C₆ cycloalkylsulfonyl, benzyl, heteroarylmethyl, pyridincarbonyl, phenylcarbonyl, tetrahydropyrancarbonyl, C₆-C₁₀ arylsulfonyl which may be optionally substituted by 1 to 2 substituents selected from the group consisting of halogen atoms, nitro (NO₂), cyano (C≡N), trifluoromethyl (CF₃), trifluoromethoxy (OCF₃), pentafluorosulfanyl (SF₅), sulfonyl (SO₃H), carboxylic group (COOH), amino (NH₂), mono-C₁-C₆ alkylamino, di-C₁-C₆ alkylamino, C₁-C₆ alkoxy, C₁-C₆ alkyl, C₁-C₆ alkoxycarbonylmethyl and phenyl which may be optionally substituted by 1 to 4 substituents selected from the group consisting of halogen atoms, C₁-C₆ acyl, nitro (NO₂), cyano (C≡N), trifluoromethyl (CF₃), trifluoromethoxy (OCF₃), pentafluorosulfanyl (SF₅), sulfonyl (SO₃H), fluorosulfonyl (SO₂F), carboxylic group (COOH), amino (NH₂), mono-C₁-C₆ alkylamino, di-C₁-C₆ alkylamino, C₁-C₆ alkoxy, C₁-C₆ alkyl, C₃-C₆ cycloalkyl and C₁-C₆ alkoxycarbonylmethyl;
   d)C₆-C₁₀ cycloalkyl which may be optionally substituted by 1 to 4 substituents selected from the group consisting of halogen atoms, C₁-C₆ acyl, nitro (NO₂), cyano (C≡N), trifluoromethyl (CF₃), trifluoromethoxy (OCF₃), pentafluorosulfanyl (SF₅), sulfonyl (SO₃H), carboxylic group (COOH), amino (NH₂), mono-C₁-C₆ alkylamino, di-C₁-C₆ alkylamino, C₁-C₆ alkoxy, C₁-C₆ alkyl, C₁-C₆ alkoxycarbonylmethyl, pyridinyloxy which may be unsubstituted or substituted by a group selected from COOH and CONHCH₃, and phenoxy which may be unsubstituted or substituted by COOH, COOR⁵, CONH₂, CN or OH;
R² is a radical selected from the group consisting of hydrogen or deuterium atoms, halogen atoms, methyl, hydroxy and C₁-C₆ alkoxy;
R³ and R⁴ are radicals which may be identical or different and which are independently selected from the group consisting of hydrogen atoms, halogen atoms, C₁-C₆ acyl, nitro (NO₂), cyano (C≡N), carboxylic group (COOH), hydroxy (OH), trifluoromethyl (CF₃), trifluoromethoxy (OCF₃), pentafluorosulfanyl (SF₅), sulfonyl (SO₃H), fluorosulfonyl (SO₂F), amino (NH₂), mono-C₁-C₆ alkylamino, di-C₁-C₆ alkylamino, C₁-C₆ alkoxy, C₁-C₆ alkyl and C₁-C₆ alkoxycarbonylmethyl;
or R³ and R⁴ may form together a radical -O-(CH₂)ₙ-O-, wherein n is an integer from 1 to 3;
R⁵ is a radical selected from C₁-C₆ alkyl and C₃-C₆ cycloalkyl.

In a particular embodiment G¹ represents a methylene group.

In a particular embodiment G¹ represents an oxygen atom.

In a particular embodiment G¹ represents a single bond.

In a particular embodiment G² represents an oxygen atom.

In a particular embodiment G³ represents a radical selected from the group consisting of -NH-(CH₂)ₘ- wherein m is an integer from 0 to 6 and -(CH₂)ₘ- wherein n is an integer from 1 to 7, more particularly G³ represents a radical-NH-(CH₂)ₘ- wherein m is an integer from 0 to 6.

In a particular embodiment when G³ is selected from the group consisting of -NH-(CH₂)ₘ- and -O-(CH₂)ₘ- wherein m has a value of 0.

In a particular embodiment when G³ is -(CH₂)ₙ- wherein n has a value of 1.

In a particular embodiment R¹ is selected from the group consisting of substituted or unsubstituted phenyl, substituted or unsubstituted cyclohexyl and substituted or unsubstituted piperidinyl. In a more specific embodiment the substituents are selected from the group consisting of methyl, trifluoromethyl, acetyl, 4-carboxy-phenoxy, isopropyl-sulfonyl, benzyl, tert-butoxycarbonyl, trifluorophenyl, propionyl, tetrahydropyran-4-carbonyl, 2-fluorobenzoyl, acetylphenyl, and 8-benzyl.

In a particular embodiment R² is selected from the group consisting of hydrogen atoms, fluorine atoms, chlorine atoms, methyl, hydroxyl and C₁-C₃ alkoxy. When G¹ represents an oxygen atom R² is preferably selected from the group consisting of hydrogen and deuterium atoms and methyl.

In another particular embodiment R² is preferably selected from the group consisting of hydrogen, methyl, hydroxyl, methoxy, fluorine and chlorine, more specifically methyl.

In a particular embodiment R³ and R⁴ are radicals which may be identical or different and which are independently selected from the group consisting of hydrogen atoms, halogen atoms, C₁-C₆ acyl, trifluoromethyl (CF₃), trifluoromethoxy (OCF₃), nitro (NO₂), amino (NH₂) and C₁-C₆ alkoxy. In a particular embodiment R³ and R⁴ may be selected from the group consisting of hydrogen, fluorine, acetyl, nitro, amino and methoxy. In a particular embodiment R³ is hydrogen and R⁴ is a radical selected from the group consisting of hydrogen atoms, halogen atoms, C₁-C₆ acyl, trifluoromethyl (CF₃), trifluoromethoxy (OCF₃), nitro (NO₂), amino (NH₂) and C₁-C₆ alkoxy.

In a particular embodiment the compound is selected from the group consisting of:
i. *p*-tolyl (9-methyl-5,6,8,9,10,11-hexahydro-7*H*-5,9:7,11-dimethanobenzo[9]annulen-7-yl)carbamate
ii. 1-(9-methyl-5,6,8,9,10,11-hexahydro-7*H*-5,9:7,11-dimethanobenzo[9]annulen-7-yl)-3-(4-(trifluoromethyl)phenyl)thiourea
iii. 1-(1-acetylpiperidin-4-yl)-3-(5-methyl-1,5,6,7-tetrahydro-1,5:3,7-dimethanobenzo[*e*]oxonin-3(2*H*)-yl)urea
iv. 1-(1-acetylpiperidin-4-yl)-3-(1,5,6,7-tetrahydro-1,5:3,7-dimethanobenzo[e]oxonin-3(2*H*)-yl)urea
v. 1-(1-acetylpiperidin-4-yl)-3-(9-methyl-5,6,8,9,10,11-hexahydro-7*H*-5,9:7,11-dimethanobenzo[9]annulen-7-yl)urea
vi. 1-(1-acetylpiperidin-4-yl)-3-(9-hydroxy-5,6,8,9,10,11-hexahydro-7*H*-5,9:7,11-dimethanobenzo[9]annulen-7-yl)urea
vii. 1-(1-acetylpiperidin-4-yl)-3-(9-methoxy-5,6,8,9,10,11-hexahydro-7*H*-5,9:7,11-dimethanobenzo[9]annulen-7-yl)urea
viii. 1-(1-acetylpiperidin-4-yl)-3-(9-fluoro-5,6,8,9,10,11-hexahydro-7*H*-5,9:7,11-dimethanobenzo[9]annulen-7-yl)urea
ix. 1-(1-acetylpiperidin-4-yl)-3-(9-chloro-5,6,8,9,10,11-hexahydro-7*H*-5,9:7,11-dimethanobenzo[9]annulen-7-yl)urea
x. 4-(((1r,4r)-4-(3-(5-methyl-1,5,6,7-tetrahydro-1,5:3,7-dimethanobenzo[*e*]oxonin-3(2*H*)-yl)ureido)cyclohexyl)oxy)benzoic acid
xi. 4-(((1r,4r)-4-(3-(9-methyl-5,6,8,9,10,11-hexahydro-7*H*-5,9:7,11-dimethanobenzo[9]annulen-7-yl)ureido)cyclohexyl)oxy)benzoic acid
xii. 1-[1-(isopropylsulfonyl)piperidin-4-yl]-3-(9-methyl-5,6,8,9,10,11-hexahydro-7*H-*5,9:7,11-dimethanobenzo[9]annulen-7-yl)urea
xiii. 1-(1-benzylpiperidin-4-yl)-3-(9-methyl-5,6,8,9,10,11-hexahydro-7*H*-5,9:7,11-dimethanobenzo[9]annulen-7-yl)urea
xiv. 1-(2-acetyl-9-methyl-5,6,8,9,10,11-hexahydro-7*H*-5,9:7,11-dimethanobenzo[9]annulen-7-yl)-3-(1-acetylpiperidin-4-yl)urea
xv. 1-(1-acetylpiperidin-4-yl)-3-(9-methyl-2-nitro-5,6,8,9,10,11-hexahydro-7*H-*5,9:7,11-dimethanobenzo[9]annulen-7-yl)urea
xvi. 1-(1-acetylpiperidin-4-yl)-3-(2-amino-9-methyl-5,6,8,910,11-hexahydro-7*H-*5,9:7,11-dimethanobenzo[9]annulen-7-yl)urea
xvii. *tert*-butyl 4-(2-((9-methyl-5,6,8,9,10,11-hexahydro-7*H*-5,9:7,11-dimethanobenzo[9]annulen-7-yl)amino)-2-oxoethyl)piperidine-1-carboxylate
xviii. *N*-(9-methyl-5,6,8,9,10,11-hexahydro-7*H*-5,9:7,11-dimethanobenzo[9]annulen-7-yl)-2-(piperidin-4-yl)acetamide
xix. 2-[1-(isopropylsulfonyl)piperidin-4-yl]-*N*-(9-methyl-5,6,8,9,10,11-hexahydro-7*H-*5,9:7,11-dimethanobenzo[9]annulen-7-yl)acetamide
xx. 2-(1-acetylpiperidin-4-yl)-*N*-(9-methyl-5,6,8,9,10,11-hexahydro-7H-5,9:7,11-dimethanobenzo[9]annulen-7-yl)acetamide
xxi. 1-(9-methyl-6,7,8,9,10,11-hexahydro-5*H*-5,9:7,11-dimethanobenzo[9]annulen-7-yl)-3-(2,3,4-trifluorophenyl)urea
xxii. 1-(5-methyl-1,5,6,7-tetrahydro-1,5:3,7-dimethanobenzo[*e*]oxonin-3(2*H*)-yl)-3-(2,3,4-trifluorophenyl)urea
xxiii. 2-(1-benzylpiperidin-4-yl)-*N*-(9-methyl-5,6,8,9,10,11-hexahydro-7*H*-5,9:7,11-dimethanobenzo[9]annulen-7-yl)acetamide
xxiv. 1-(9-methyl-5,6,8,9,10,11-hexahydro-7*H*-5,9:7,11-dimethanobenzo[9]annulen-7-yl)-3-(1-propionylpiperidin-4-yl)urea
xxv. 1-(1-(4-acetylphenyl)piperidin-4-yl)-3-(9-methyl-5,6,8,9,10,11-hexahydro-7*H-*5,9:7,11-dimethanobenzo[9]annulen-7-yl)urea
xxvi. 1-(9-methyl-5,6,8,9,10,11-hexahydro-7*H*-5,9:7,11-dimethanobenzo[9]annulen-7-yl)-3-(1-(tetrahydro-2*H*-pyran-4-carbonyl)piperidin-4-yl)urea
xxvii. 1-(1-(2-fluorobenzoyl)piperidin-4-yl)-3-(9-methyl-5,6,8,9,10,11-hexahydro-7*H-*5,9:7,11-dimethanobenzo[9]annulen-7-yl)urea
xxviii. 1-((1R,3s,5S)-8-benzyl-8-azabicyclo[3.2.1]octan-3-yl)-3-(9-methyl-5,6,8,9,10,11-hexahydro-*7H*-5,9:7,11-dimethanobenzo[9]annulen-7-yl)urea
xxix. 1-(1-acetylpiperidin-4-yl)-3-(2-fluoro-9-methyl-5,6,8,9,10,11-hexahydro-*7H-*5,9:7,11-dimethanobenzo[9]annulen-7-yl)urea
xxx. 1-(1-acetylpiperidin-4-yl)-3-(2-methoxy-9-methyl-5,6,8,9,10,11-hexahydro-*7H-*5,9:7,11-dimethanobenzo[9]annulen-7-yl)urea
xxxi. 1-(1-acetylpiperidin-4-yl)-3-(1-fluoro-9-methyl-5,6,8,9,10,11-hexahydro-*7H-*5,9:7,11-dimethanobenzo[9]annulen-7-yl)urea
xxxii. 1-(1-acetylpiperidin-4-yl)-3-(2,3-dimethoxy-9-methyl-5,6,8,9,10,11-hexahydro-*7H-*5,9:7,11-dimethanobenzo[9]annulen-7-yl)urea
xxxiii. 1-(1-acetylpiperidin-4-yl)-3-(5,8,9,10-tetrahydro-5,8:7,10-dimethanobenzo[8]annulen-7(*6H*)-yl)urea
xxxiv. 1-(benzo[*d*]thiazol-2-yl)-3-(9-methoxy-5,6,8,9,10,11-hexahydro-7*H*-5,9:7,11-dimethanobenzo[9]annulen-7-yl)urea

Another aspect of the present invention relates to pharmaceutical or veterinary compositions comprising therapeutically effective amounts of compounds of formula (I), or stereoisomers or pharmaceutically acceptable salts thereof, and preferably adequate amounts of pharmaceutically acceptable excipients. Pharmacy in the context of the present invention relates both to human medicine and veterinary medicine.

Another aspect of the present invention relates to compounds of formula (I), or stereoisomer or pharmaceutically acceptable salts thereof, for use as a medicament.

In a particular embodiment the present invention relates to compounds of formula (I), or stereoisomer or pharmaceutically acceptable salts thereof, for use in the treatment or prevention in an animal, including a human, of a disease or disorder susceptible of improvement by inhibition of soluble epoxide hydrolase.

In particular embodiments the disease or disorder susceptible of improvement by inhibition of soluble epoxide hydrolase are selected from the group consisting of hypertension, atherosclerosis, pulmonary diseases, kidney diseases, stroke, pain, neuropathic pain, inflammation, pancreatitis in particular acute pancreatitis, immunological disorders, eye diseases in particular diabetic keratopathy, wet age-related macular degeneration and retinopathy such as premature retinopathy and diabetic retinopathy, cancer, obesity, including obesity-induced colonic inflammation, diabetes, metabolic syndrome, preeclampsia, anorexia nervosa, depression, erectile dysfunction, wound healing, NSAID-induced ulcers, emphysema, scrapie, Parkinson's disease, arthritis, arrhythmia, Alzheimer's disease, Raynaud's syndrome, cardiomyopathy, vascular cognitive impairment, mild cognitive impairment, inflammatory bowel diseases, cirrhosis, osteoporosis, chronic periodontitis, sepsis, seizure disorders (epilepsy), dementia, edema, attention-deficit hyperactivity disorder, schizophrenia, drug dependency, social anxiety, colitis, amyotrophic lateral sclerosis, chemotherapy induced side effects, laminitis, inflammatory joint pain and synovitis, endothelial dysfunction, subarachnoid hemorrhage, including aneurysmal subarachnoid hemorrhage, traumatic brain injury and diabetes-induced learning and memory impairment.

In other words, the present invention is related to methods of treatment or prevention in an animal, including a human, of a disease or disorder susceptible of improvement by inhibition of soluble epoxide hydrolase, by administration of pharmaceutical or veterinary compositions comprising compounds of formula (I). Methods for treatment of the aforementioned particular diseases and disorders are particular embodiments of the present invention.

According to another aspect of the present invention, the compounds of formula (Ia) wherein G² is oxygen, G³ is -NH-(CH₂)ₘ- may be prepared by reacting the amine of formula (II), preferably in the form of a salt such as the hydrochloride with isocyanate of formula (III), in an inert solvent such as dichloromethane (DCM), and in the presence of a base such as triethylamine.

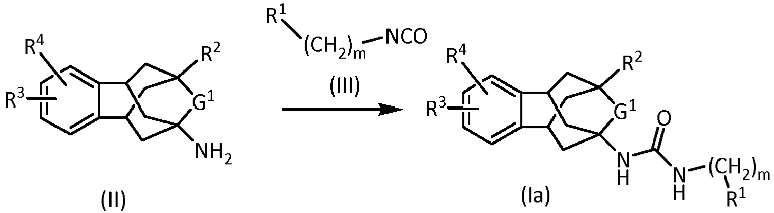

According to another aspect of the present invention, the compounds of formula (Ia), wherein G² is oxygen, G³ is -NH-(CH₂)ₘ₋, may also be prepared by converting in a first step the amine of formula (II), preferably in the form of a salt, into isocyanate of formula (IV) by reaction with an (NH₂→NCO)-converting reagent, such as triphosgene, in an inert solvent, such as DCM. In a second step, the amine of formula (V) is reacted with the isocyanate of formula (IV) to yield compound of formula (Ia). The coupling reaction may be carried out without catalyst and the reaction conveniently takes place at room temperature in the presence of an organic solvent, typically DCM, tetrahydrofurane (THF) or *N,N*-dimethylformamide (DMF).

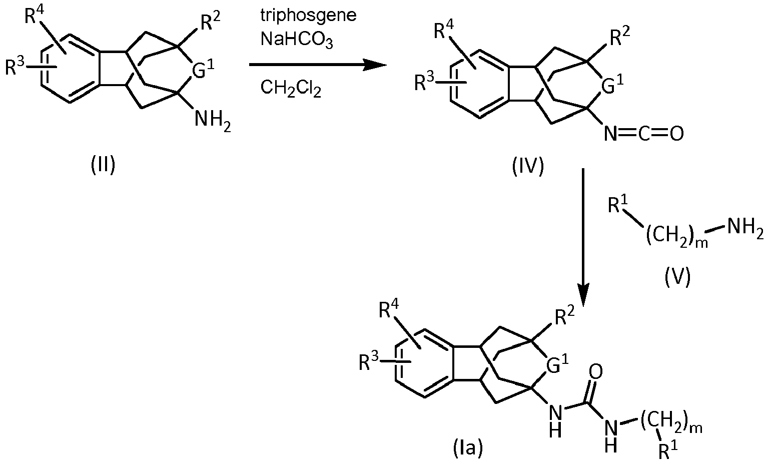

According to another aspect of the present invention, the compounds of formula (Ib), wherein G² is sulfur, G³ is -NH-(CH₂)ₘ₋, may be prepared by converting in a first step the amine of formula (II) preferably in the form of a salt, into a dithiocarbamate salt of formula (VI) by reaction with carbon disulfide in an inert solvent, such as THF, in the presence of a base, such as triethylamine. In a second step, the dithiocarbamate salt is decomposed in the presence of tosyl chloride to yield the isothiocyanate of formula (VII) which is subsequently reacted with an amine of formula R¹-(CH₂)ₘ-NH₂ of formula (V) to yield compound of formula (Ib).

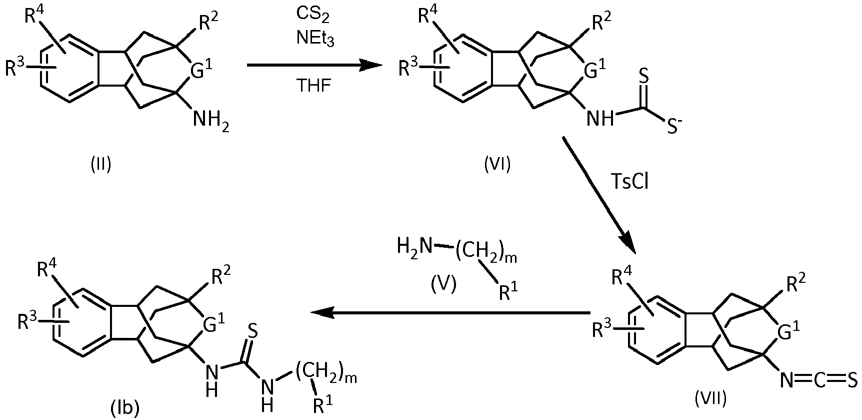

According to another aspect of the present invention, the compounds of formula (Ib) wherein G² is sulphur and G³ is -NH-(CH₂)ₘ- may also be prepared by reacting the amine of formula (II), preferably in the form of a salt such as the hydrochloride with thioisocyanate of formula SCN-(CH₂)ₘ-R¹ (VIII), in an inert solvent, such as DCM, and in the presence of a base such as triethylamine.

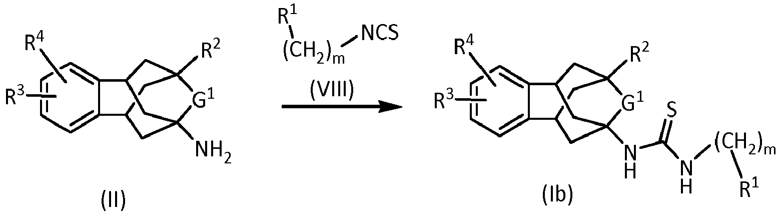

According to another aspect of the present invention, the compounds of formula (Ic), wherein G² and G³ are both oxygen, may be prepared by reacting the amine of formula (II) with the chloroformate of formula (IX) in the presence of a base such as triethylamine.

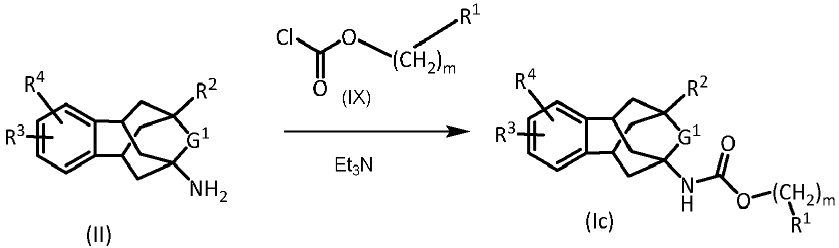

According to another aspect of the present invention, the compounds of formula (Id), wherein G² is oxygen and G³ is -(CH₂)ₙ-, may be prepared by reacting the amine of formula (II), preferably in the form of a salt such as the hydrochloride, with a carboxylic acid of formula (X) in the presence of a coupling agent such as EDCI or HOBt or using an acyl chloride in the presence of a base, such as triethylamine, in an organic solvent such as ethyl acetate.

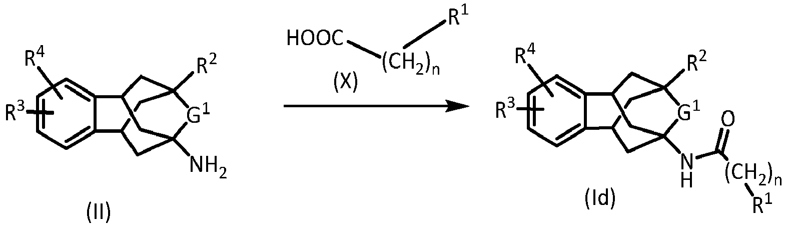

The amines of formula (II) may be obtained using a range of different reactions depending on the nature of the substituents G¹, R², R³ and R⁴ and some amines of formula (II) are disclosed in the art (see for example Bioorg Med Chem. 2010, 18, 46; Bioorg Med Chem. 2012, 20, 942; Bioorg Med Chem. 2014, 22, 2678; Bioorg Med Chem. 2015, 23, 290).

When G¹ is CH₂ and R² is OH the amines of formula (IIa) may be prepared according to the reaction scheme shown below:

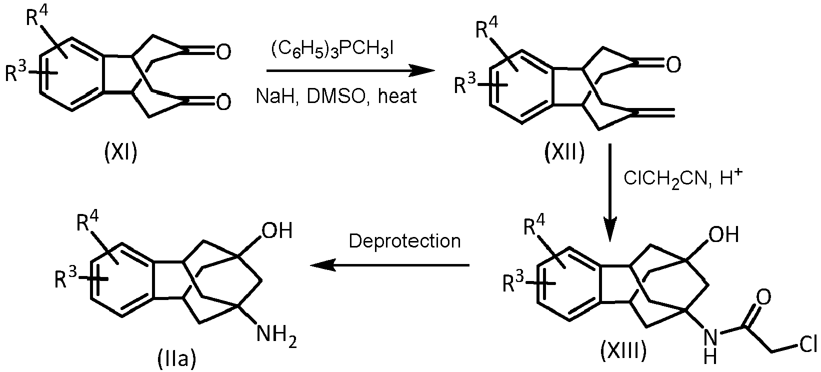

The deprotection step of the chloroacetamide to yield the final amine (IIa) may be carried out by refluxing overnight the compound (XIII) in the presence of thiourea and acetic acid in ethanol.

Diketone (XI) is a known compound when R³ = R⁴ = H (Liebigs Ann Chem. 1973; 1839-1850). In general, substituted diketones of formula (XI) may be prepared from substituted o-phthalaldehydes (XIV) according to the reaction scheme shown below.

Starting from suitably substituted o-phthalaldehyde derivatives of formula (XIV) and 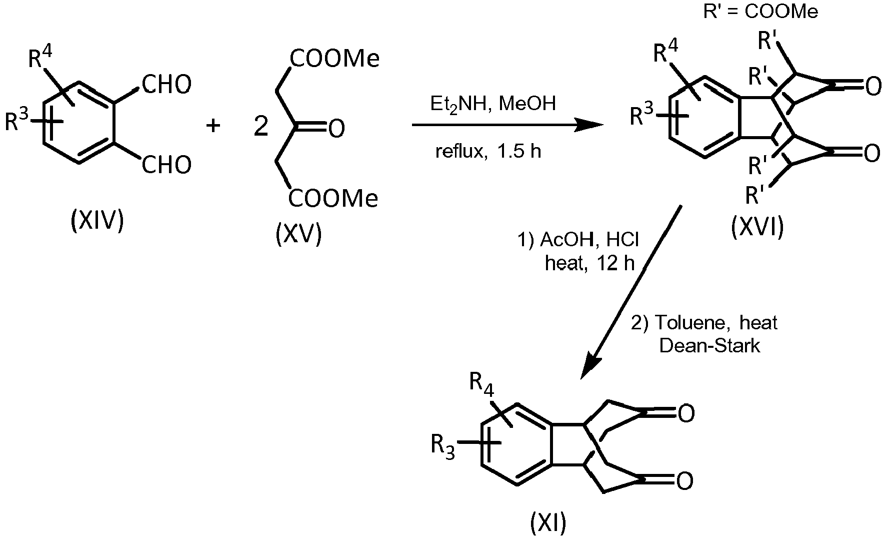 following the reaction scheme shown above, it is also possible to prepare diketones (XI) with different substituents such as those shown below:

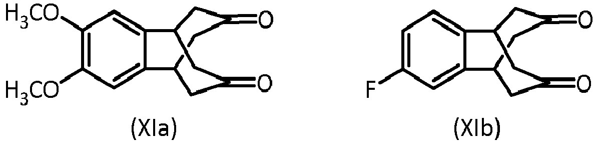

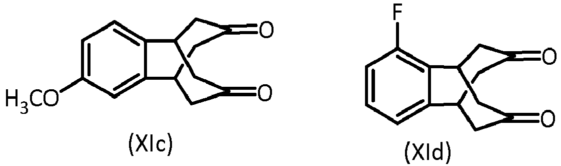

When G¹ is CH₂ and R² is C₁-C₆ alkoxy the amines of formula (IIb) may be prepared according to the reaction scheme shown below: 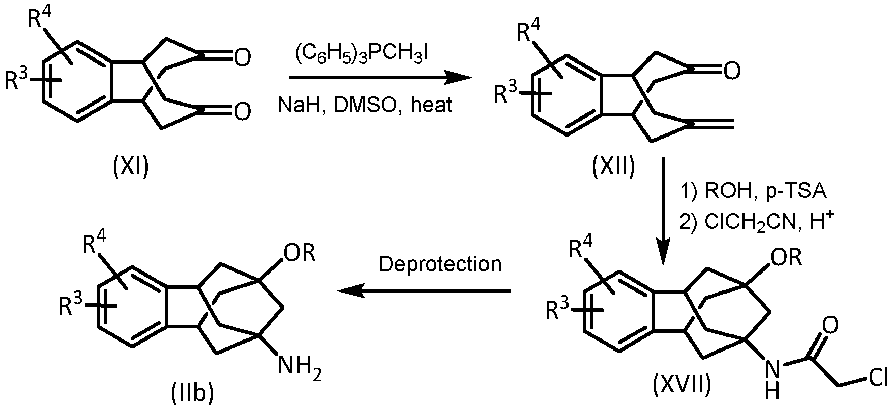 wherein R is C₁₋₆ alkyl

The deprotection step of the chloroacetamide to yield the final amine (IIb) may be carried out by refluxing overnight the compound (XVII) in the presence of thiourea and acetic acid in ethanol.

When G¹ is CH₂ and R² is methyl the amines of formula (IIc) may be prepared according to the reaction scheme shown below:

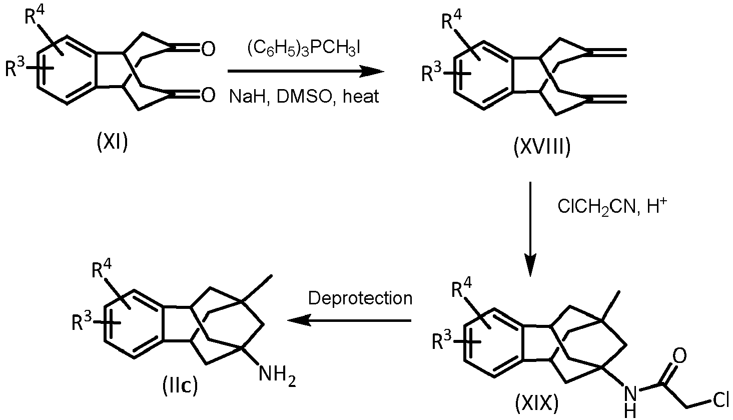

The deprotection step of the chloroacetamide to yield the final amine (IIc) may be carried out by refluxing overnight the compound (XIX) in the presence of thiourea and acetic acid in ethanol.

When G¹ is CH₂ and R² is bromine or fluorine the amines of formula (IId) and (IIe) may be prepared according to the reaction scheme shown below:

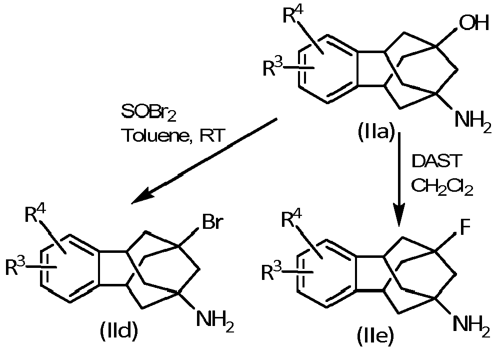

Alternatively, the amine (IIe) may be obtained starting from compound (XIII) according to the scheme below:

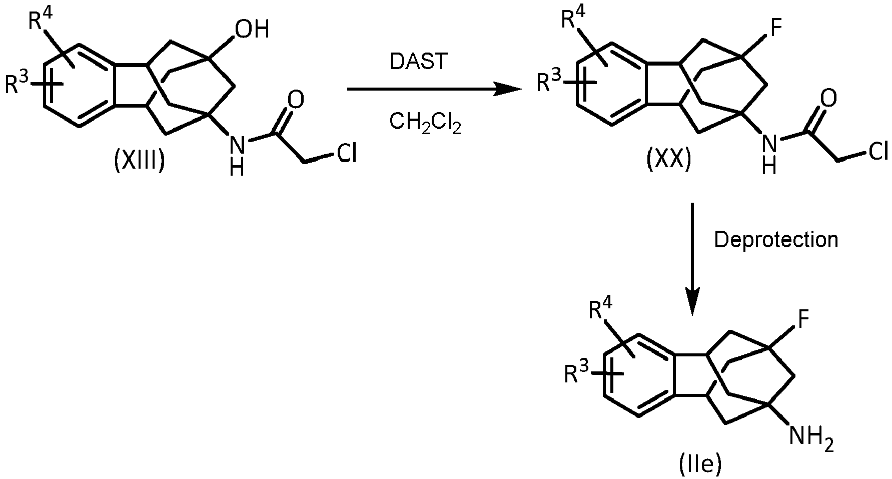

The deprotection step of the chloroacetamide to yield the final amine (IIe) may be carried out by refluxing overnight the compound (XX) in the presence of thiourea and acetic acid in ethanol.

When G¹ is CH₂ and R² is chlorine the amines of formula (IIf) may be prepared according to the reaction scheme shown below:

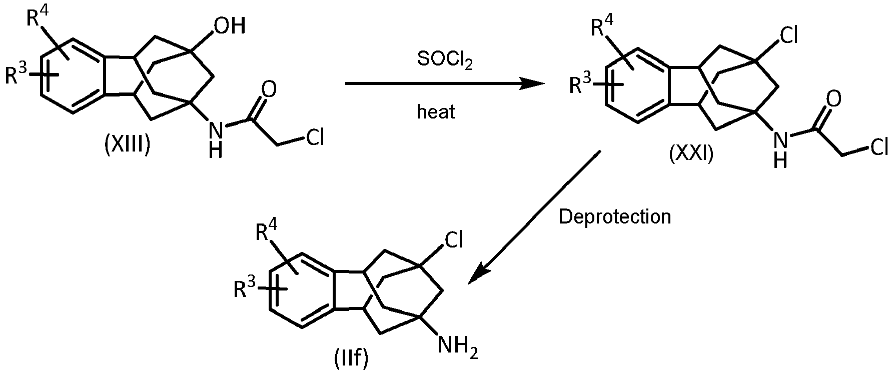

The deprotection step of the chloroacetamide to yield the final amine (IIf) may be carried out by refluxing overnight the compound (XXI) in the presence of thiourea and acetic acid in ethanol.

When G¹ is CH₂ and R² is hydrogen the amines of formula (IIg) may be prepared according to the reaction scheme shown below:

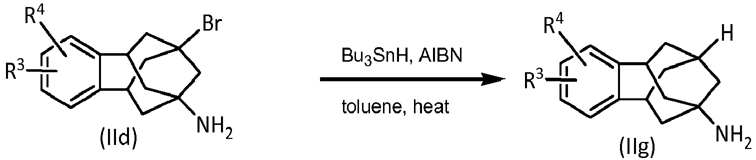

When G¹ is CH₂ and R² is deuterium the amines of formula (IIh) may be prepared according to the reaction scheme shown below:

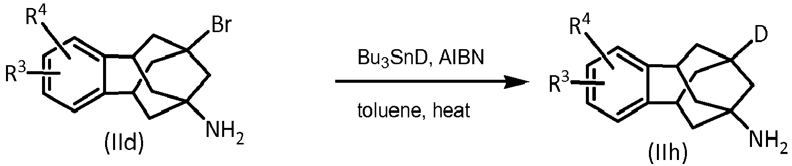

When G¹ is O and R² is methyl the amines of formula (IIi) may be prepared according to the reaction scheme shown below:

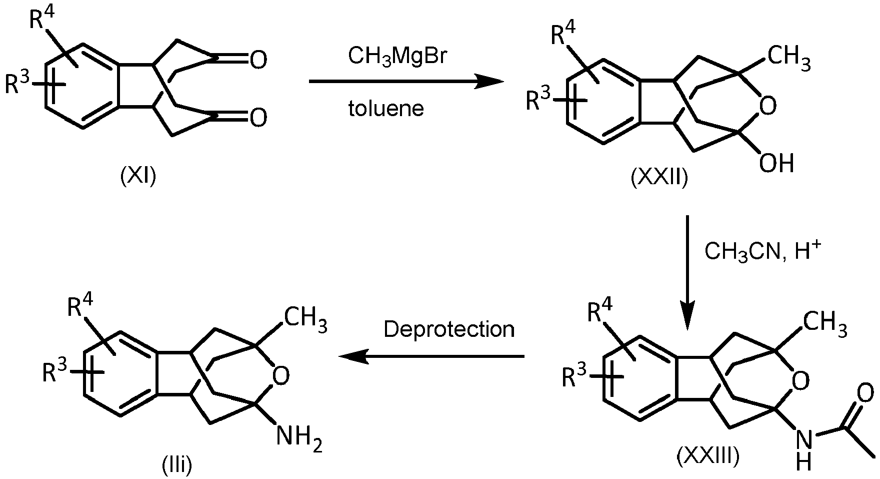

The deprotection step of the acetamide to yield the final amine (IIi) may be carried out by refluxing overnight the compound (XXIII) in the presence of conc. HCl as reported for R³ = R⁴ = H in Bioorg Med Chem 2010, 18, 46-57.

Alternatively, when G¹ is O and R² is methyl the amines of formula (IIi) may be prepared according to the reaction scheme shown below:

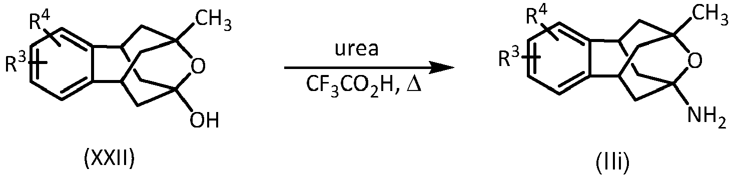

When G¹ is O and R² is hydrogen the amines of formula (IIj) may be prepared according to the reaction scheme shown below:

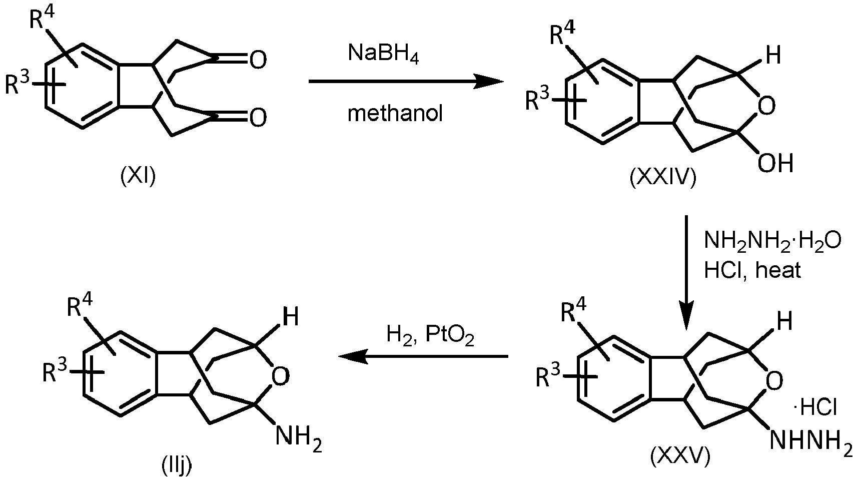

When G¹ is O and R² is deuterium the amines of formula (IIk) may be prepared according to the reaction scheme shown below:

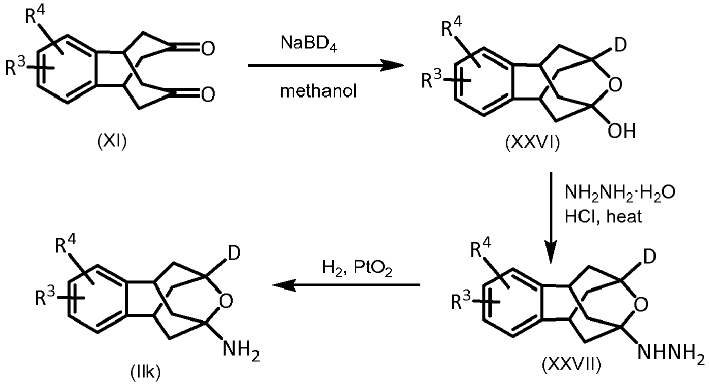

When G¹ is O and R² is a halogen or a hydroxyl group the compounds of formula (Ie) and (If) may be prepared according to the reaction scheme shown below:

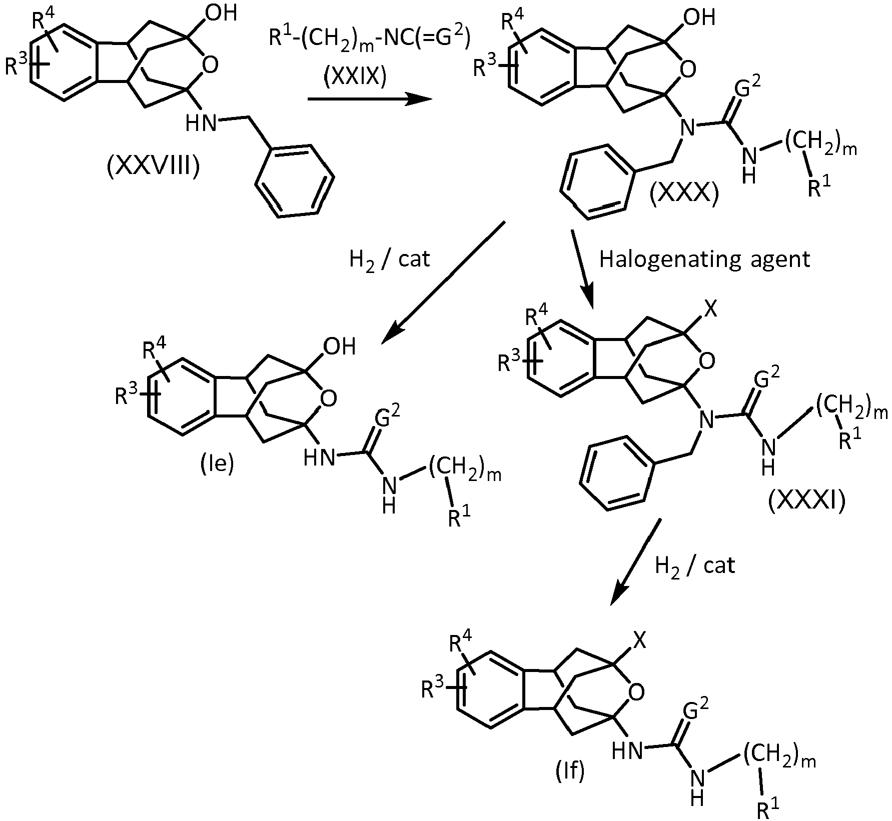

When X is fluorine in the second step compound (XXX) is converted to compound (XXXI) using (diethylamino)sulfur trifluoride (DAST) as halogenating agent, when X is chlorine the halogenating agent is SOCl₂ and when X is bromine the halogenating agent is SOBr₂.

The preparation of compound (XXVIII) is described from compound (XI) (for R³=R⁴=H) in patent application DE 2 210 799 A1. The synthetic process described therein may be also used for the preparation of compounds where R³ and/or R⁴ are different from hydrogen.

When G¹ is a bond and R² is a fluorine the compounds of formula (IIn) may be prepared according to the reaction scheme shown below:

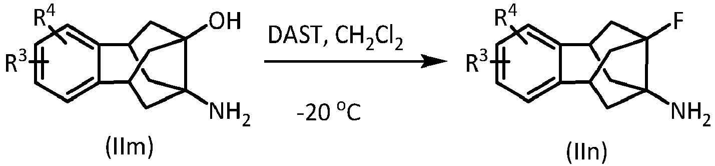

The preparation of compound (IIm) is described (for R³=R⁴=H) in Liebigs Ann. 1995, 523-535. The synthetic process described therein may be also used for the preparation of compounds where R³ and/or R⁴ are different from hydrogen.

Compounds of formula (IIo) and (IIp) may be prepared, respectively, from compounds (XXXII) and (XXXIII) through one or more well-known reactions. Compounds (XXXII) and (XXXIII) are synthesized from compounds of formula (XI) according to methods reported in the literature for R³=R⁴=H (Liebigs Ann Chem. 1973; 1839-1850 and Aust J Chem. 1983, 36, 2465-2472) which methods may also be used for the preparation of compounds where R³ and/or R⁴ are different from hydrogen.

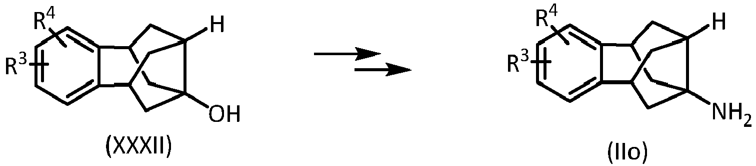

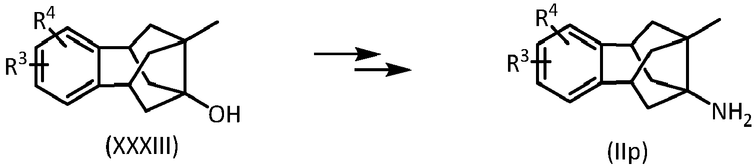

It is also possible to convert some compounds of formula (II) to other compounds of formula (II) by modifying the nature of the groups R³ and R⁴ by conventional methods known to the person skilled in the art. As an example, compound of formula (IIq) may be converted to compound of formula (IIr) by catalytic hydrogenation.

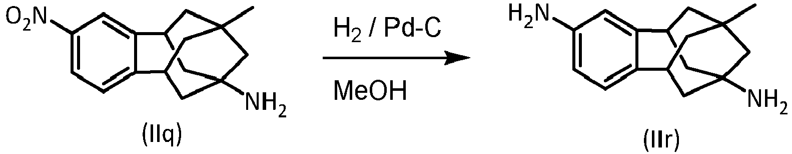

It is worth mentioning that it is possible to convert some compounds of formula (I) of the invention to other compounds of formula (I) by modifying the nature of the groups R³ and R⁴ by conventional methods known to the person skilled in the art. As an example, compounds of formula (I) wherein R³ and R⁴ are hydrogen atoms may be converted to compounds where one R³ and R⁴ is hydrogen and the other one is a C₁₋₆ acyl group through a Friedel-Craft reaction. As another example compounds of formula (I) wherein R³ and/or R⁴ are a nitro group may be converted to compounds where said R³ and/or R⁴ is an amino group by catalytic hydrogenation.

Finally, it is worth mentioning that the compounds of the invention may also be prepared following the methods explained above from precursors of formula (XXXIV) wherein the rest R⁶ is a precursor of the rest R¹ which is converted into said rest R¹ through one or more well-known reactions. It is also possible that the rest R⁶ is already a group R¹ which is converted into another group R¹ through one or more well-known reactions.

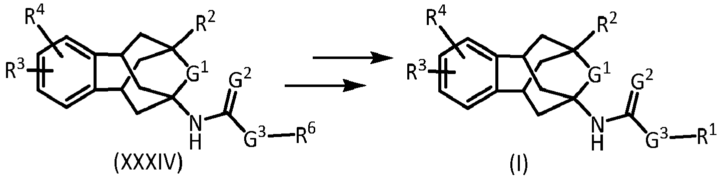

Examples of said synthetic strategy are provided below wherein the group R⁶ is an unsubstituted piperidinyl rest and R¹ is a piperidinyl rest carrying substituents as defined in the claims:

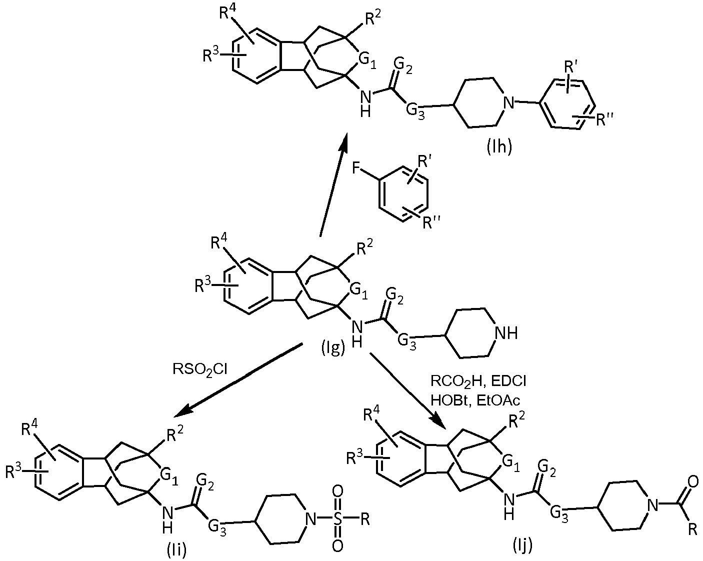

The reaction of compound (Ig) to yield compound (Ih) is carried out using K₂CO₃ and anhydrous DMSO applying heat. The reaction of compound (Ig) to yield compound (Ij) is carried out either as shown (RCO₂H, EDCI, HOBt, EtOAc) or using RCOCI and Et₃N in DCM.

In addition to the three kind of derivatives shown in the scheme above, it is also possible to go from the unsubstituted piperidine in (Ig) to benzyl piperidines. The procedure involves the reaction of the piperidine (Ig) with benzaldehydes and sodium cyanoborohydride in acetic acid / methanol.

As used herein, the term methylene designates the radical -(CH₂)-.

As used herein the term aryl designates an aromatic carbocyclic ring which may be unsubstituted or substituted. Non-limiting examples of unsubstituted aryl groups are phenyl and anthranyl.

As used herein the term halogen atoms designates atoms selected from the group consisting of chlorine, fluorine, bromine and iodine atoms, preferably fluorine, chlorine or bromine atoms. The term halo when used as a prefix has the same meaning.

As used herein the term Cₚ acyl designates a group alkyl having p-1 carbon atoms which is linked to a carbonyl group (CH₃-(CH₂)ₚ₋₂-CO-). Non limiting examples of acyl groups are acetyl, propionyl, butyryl, valeryl and caproyl.

As used herein the term C_{q} alkyl designates linear or branched hydrocarbon radicals (C_{q}H_{2q+1}-). Non-limiting examples of alkyl groups are methyl, ethyl, *n*-propyl *i*-propyl, *n-*butyl, *i*-butyl, *sec*-butyl, *tert*-butyl, *n*-pentyl, *i*-pentyl and *n*-hexyl.

As used herein the term mono-Cᵣ-alkylamino designates a Cᵣ-alkyl linked to a group NH (Cᵣ-alkyl-NH-). Non-limiting examples of monoalkylamino groups are methylamino (CH₃-NH-), ethylamino (CH₃-CH₂-NH-) and *n*-propylamino (CH₃-CH₂-CH₂-NH-).

As used herein the term di-Cₛ-alkylamino designates two alkyl rest linked to a group N ((Cₛ-alkyl)₂-N-) wherein the two alkyl rests may have the same or different number of carbon atoms. Non-limiting examples of dialkylamino groups are dimethylamino ((CH₃)₂NH-), diethylamino ((CH₃-CH₂)₂N-), ethylmethylamino ((CH₃)(CH₃-CH₂)N-) and di-n-propylamino ((CH₃-CH₂-CH₂)₂N-).

As used herein the term Cₜ alkoxy designates a linear or branched alkyl group linked to an oxygen atom (CH₃-(CH₂)ₜ₋₁-O-). Non-limiting examples of alkoxy groups are methoxy, ethoxy, *n*-propoxy, *i*-propoxy, *n*-butoxy, *i*-butoxy, *sec*-butoxy, *tert*-butoxy, *n-*pentoxy, *i*-pentoxy and *n*-hexoxy.

As used herein the term Cᵤ alkoxycarbonylmethyl designates a Cᵤ alkoxy rest linked to a group -CO-CH₂- ((CH₃-(CH₂)ᵤ₋₁)-O-CO-CH₂-). Non-limiting examples of alkoxycarbonylmethyl groups are methoxycarbonylmethyl and ethoxycarbonylmethyl.

As used herein the term methylaminocarbonylpyridyloxy is used to designate the group:

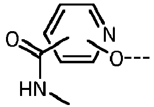

As used herein the term heteroaryl designates an heteroaromatic ring containing carbon, hydrogen and one or more heteroatoms selected from N, O and S as part of the ring. Said radicals may be unsubstituted or substituted by one or more substituents. Non-limiting examples of heteroaryl groups are pyridyl, pyrimidinyl, furyl, thienyl, pyrazolyl, oxazolyl and thiazolyl.

As used herein the term saturated or partially unsaturated heterocyclyl is used to designate a non-aromatic ring containing carbon, hydrogen and one or more heteroatoms selected from N, O and S as part of the ring. In particular, an heterocyclyl group may be monocyclic or bicyclic. Non-limiting examples of saturated heterocyclyl groups are piperidinyl, morpholinyl, tetrahydropyranyl and piperazinyl.

As used herein the term cycloalkyl designates hydrocarbon cyclic groups. Said cycloalkyl groups may have a single cyclic ring or a polycyclic ring. Non-limiting examples of cycloalkyl groups are cyclopropyl, cyclobutyl, cyclopentyl and cyclohexyl.

As used herein the term alkylsulfonyl designates a linear or branched alkyl group linked to a sulfonyl group (CH₃-(CH₂)ᵥ₋₁-SO₂-). Non-limiting examples of alkylsulfonyl groups are methylsulfonyl (CH₃-SO₂-), ethylsulfonyl (CH₃-CH₂-SO₂-) and *n*-propylsulfonyl (CH₃-CH₂-CH₂-SO₂-).

As used herein the term cycloalkylsulfonyl designates a cycloalkyl group linked to a sulfonyl group. Non-limiting examples of cycloalkylsulfonyl groups are cyclopropylsulfonyl, cyclobutylsulfonyl, cyclopentylsulfonyl and cyclohexylsulfonyl.

As used herein the term arylsulfonyl designates an aryl group linked to a sulfonyl group. Non-limiting examples of alkylsulfonyl groups are phenylsulfonyl and naphthalenesulfonyl.

As used herein the term pyridincarbonyl designates a pyridyl group linked to a carbonyl group (C₅H₄N-CO-).

As used herein the term phenylcarbonyl designates a phenyl group linked to a carbonyl group (C₆H₅-CO-).

As used herein the term tetrahydropyrancarbonyl designates a tetrahydropyranyl group linked to a carbonyl group (C₅H₉O-CO-).

As used herein the term pharmaceutically acceptable salt designates any salt which, upon administration to the patient is capable of providing (directly or indirectly) a compound as described herein. For instance, pharmaceutically acceptable salts of compounds provided herein are synthesized from the parent compound, which contains a basic or acidic moiety, by conventional chemical methods. Generally, such salts are, for example, prepared by reacting the free acid or base forms of these compounds with a stoichiometric amount of the appropriate base or acid in water or in an organic solvent or in a mixture of both. Generally, non-aqueous media like ether, ethyl acetate, ethanol, 2-propanol or acetonitrile are preferred. Examples of the acid addition salts include mineral acid addition salts such as, for example, hydrochloride, hydrobromide, hydroiodide, sulfate, nitrate, phosphate, and organic acid addition salts such as, for example, acetate , trifluoroacetate, maleate , fumarate , citrate , oxalate , succinate, tartrate, malate, mandelate, methanesulfonate and *p*-toluenesulfonate. Examples of the alkali addition salts include inorganic salts such as, for example, sodium, potassium, calcium and ammonium salts, and organic alkali salts such as, for example, ethylenediamine, ethanolamine, *N,N*-dialkylenethanolamine, triethanolamine and basic aminoacids salts.

As used herein the term stereoisomers designates molecules that have the same molecular formula and sequence of bonded atoms (constitution), but differ in the three-dimensional orientations of their atoms in space. The compounds of formula (I) have at least two chiral carbon atoms (marked as 1 and 3 in the formula depicted below) and, thus, several stereoisomers of said compounds may exist. Said stereoisomers are encompassed by formula (I).

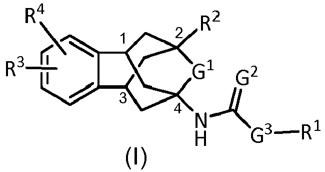

Throughout the description and claims the word "comprise" and variations of the word, are not intended to exclude other technical features, additives, components, or steps. Furthermore, the word "comprise" encompasses the case of "consisting of". Additional objects, advantages and features of the invention will become apparent to those skilled in the art upon examination of the description or may be learned by practice of the invention. The following examples are provided by way of illustration, and they are not intended to be limiting of the present invention. Furthermore, the present invention covers all possible combinations of particular and preferred embodiments described herein.

### ABREVIATIONS:

The following abreviations have been used along the present application:
- anh.:: anhydrous
- AcOH:: acetic acid
- AcCl:: acetyl chloride
- AIBN:: azobisisobutyronitrile
- Bis/Tris:: 2-Bis(2-hydroxyethyl)amino-2-(hydroxymethyl)-1,3-propanediol
- BSA:: bovine serum albumin
- Bu₃SnD:: tributyl(deuterio)stannane
- Calcd:: calculated
- d:: doublet
- DAST:: diethylaminosulfur trifluoride
- Dec:: decomposes
- DCM:: dichloromethane
- DMF:: *N,N*-dimethylformamide
- DMSO:: dimethylsulfoxide
- dq: doublet of quartets
- dt: doublet of triplets
- EDCI:: 1-ethyl-3-(3-dimethylaminopropyl)carbodiimide
- ESI:: electrospray ionization
- Et₂O:: diethylether
- Et₃N:: triethylamine
- EtOAc:: ethyl acetate
- EtOH:: ethanol
- HOBt:: hydroxybenzotriazole
- h:: hours
- Hz: Hertz
- HRMS:: high resolution mass spectroscopy
- IR:: infrared
- m:: multiplet
- MeOH:: methanol
- mp:: melting point
- *n*-Bu:: *n*-butyl
- NMR:: nuclear magnetic resonance
- NSAID:: non steroidal anti-inflammatory drug
- *p*-TSA:: *p*-toluenesulfonic acid
- PHOME:: cyano(6-methoxynaphthalen-2-yl)methyl 2-(3-phenyloxiran-2-yl)acetate
- s:: singlet
- sEH:: soluble epoxide hydrolase
- t:: triplet
- THF:: tetrahydrofurane
- TPPU:: *N*-[1-(1-Oxopropyl)-4-piperidinyl]-*N*'-[4-(trifluoromethoxy)phenyl]urea
- UV:: ultraviolet

### EXAMPLES

### Analytical methods

- Melting points were determined in open capillary tubes with a MFB 595010 M Gallenkamp melting point apparatus.
- Infrared (IR) spectra were run either on a Perkin-Elmer Spectrum RX I spectrophotometer (using the attenuated total reflectance technique) or on a spectrophotometer Nicolet Avatar 320 FT-IR. Absorption values are expressed as wavenumbers (cm⁻¹); only significant absorption bands are given.
- Elemental analyses were carried out at the Mycroanalysis Service of the IIQAB (CSIC, Barcelona, Spain) with a Carlo Erba model 1106 analyzer.
- Preparative normal phase chromatography was performed on a CombiFlash Rf 150 (Teledyne Isco) with pre-packed RediSep Rf silica gel cartridges. Thin-layer chromatography was performed with aluminum-backed sheets with silica gel 60 F254 (Merck, ref 1.05554 or Sigma-Aldrich, ref 60805), and spots were visualized with UV light, 1% aqueous solution of KMnO₄ and/or iodine.
- High-resolution mass spectrometry (HRMS) analyses were performed with an LC/MSD TOF Agilent Technologies spectrometer.
- Analytical grade solvents were used for crystallization, while pure for synthesis solvents were used in the reactions, extractions and column chromatography.
- The analytical samples of all of the new compounds which were subjected to pharmacological evaluation possess a purity ≥95% as evidenced by their elemental analysis.

### Reference example 1: 2-fluoro-5,6,8,9-tetrahvdro-7H-5,9-propanobenzo[7]annulene-7,11-dione.

In a round-bottomed flask equipped with a condenser and magnetic stirring a solution of 4-fluorophthalaldehyde (3.08 g, 20 mmol) and dimethyl 3-oxopentanedioate (6.98 g, 40 mmol) in MeOH (60 mL) was prepared. Four drops of diethylamine were added and the reaction was heated at reflux for 1.5 h, the reaction was cooled down and 7 drops more of diethylamine were added and the reaction was stored at 4°C overnight. The precipitate was filtered off under vacuum and was washed with cold MeOH (4 mL), obtaining tetramethyl 2-fluoro-7,11-dioxo-6,7,8,9-tetrahydro-5*H*-5,9-propanobenzo[7] annulene-6,8,10,12-tetracarboxylate as white needles (3.05 g). A solution of this solid in glacial acetic acid (18 mL) and conc. HCl (5 mL) was heated at reflux for 12 h. The solvent was removed under vacuum to give a solid. A solution of this solid in toluene (50 mL) was heated a reflux for 16 h in a Dean-Stark apparatus. The toluene was removed under vacuum to give pure 2-fluoro-5,6,8,9-tetrahydro-7*H*-5,9-propanobenzo[7]annulene-7,11-dione (1.53 g, 33% overall yield) as a light brown solid.
mp 105-107 °C. IR (NaCl disk): 2923, 2848, 1710, 1607, 1593, 1490, 1428, 1380, 1346, 1253, 1208, 1119, 1074, 985, 944, 865, 806 cm⁻¹. HRMS-ESI+ m/z [M+H]⁺ calcd for [C₁₄H₁₃FO₂+H]⁺: 233.0972, found: 233.0967.

### Reference example 2: 2-methoxy-5,6,8,9-tetrahydro-7H-5,9-propanobenzo[7]annulene-7,11-dione:

From 4-methoxyphthalaldehyde (10.2 g, 61.9 mmol), dimethyl 3-oxopentanedioate (21.5 g, 124 mmol) and diethylamine (28 drops) in MeOH (380 mL) and following the procedure described in reference example 1, tetramethyl 2-methoxy-7,11-dioxo-6,7,8,9-tetrahydro-5*H*-5,9-propanobenzo[7]annulene-6,8,10,12-tetracarboxylate was obtained (19.4 g, 66% yield). From the aforementioned tetracarboxylate (250 mg, 0.5 mmol), conc. HCl (0.4 mL) and glacial acetic acid (1.4 mL) and following the procedure described in reference example 1, 2-methoxy-5,6,8,9-tetrahydro-7*H*-5,9-propanobenzo[7]annulene-7,11-dione (125 mg, 98% yield) was obtained.
mp 157-158 °C. IR (NaCl disk): 2941, 2910, 2837, 1701, 1610, 1585, 1504, 1431, 1414, 1370, 1321, 1300, 1266, 1166, 1094, 1033, 989 cm⁻¹. HRMS-ESI+ m/z [M+H]⁺ calcd for [C₁₅H₁₆O₃+H]⁺: 245.1172, found: 245.1180.

### Reference example 3: 2,3-dimethoxy-5,6,8,9-tetrahydro-7H-5,9-propanobenzo [7]annulene-7,11-dione:

From 4,5-dimethoxyphthalaldehyde (6.54 g, 33.7 mmol), dimethyl 3-oxopentanedioate (11.7 g, 67.4 mmol) and diethylamine (19 drops) in MeOH (130 mL) and following the procedure described in reference example 1, tetramethyl 2,3-dimethoxy-7,11-dioxo-6,7,8,9-tetrahydro-5*H*-5,9-propanobenzo[7]annulene-6,8,10,12-tetracarboxylate was obtained (6.84 g, 40% yield). From the aforementioned tetracarboxylate (6.84 g, 13.5 mmol), conc. HCl (10 mL) and glacial acetic acid (35 mL), and following the procedure described in reference example 1, 2,3-dimethoxy-5,6,8,9-tetrahydro-7*H*-5,9-propanobenzo [7]annulene-7,11-dione (2.8 g, 76% yield) was obtained.
mp 236-237 °C. IR (NaCl disk): 2952, 2840, 1698, 1605, 1516, 1467, 1451, 1416, 1355, 1336, 1254, 1221, 1192, 1162, 1025, 1002, 880, 811 cm⁻¹. HRMS-ESI+ m/z [M+H]⁺ calcd for [C₁₆H₁₈O₄+H]⁺: 275.1278, found: 275.1279.

### Reference example 4: 1-fluoro-5,6,8,9-tetrahydro-7H-5,9-propanobenzo[7]annulene-7,11-dione.

From 3-fluorophthalaldehyde (11.2 g, 73.6 mmol), dimethyl 3-oxopentanedioate (25.6 g, 147 mmol) and diethylamine (33 drops) in MeOH (220 mL) and following the procedure described in reference example 1, tetramethyl 1-fluoro-7,11-dioxo-6,7,8,9-tetrahydro-5*H*-5,9-propanobenzo[7]annulene-6,8,10,12-tetracarboxylate was obtained (18.5 g, 54% yield). The aforementioned tetracarboxylate (18.5 g, 39.9 mmol), conc. HCl (31 mL) and glacial acetic acid (103 mL), and following the procedure described in reference example 1, 1-fluoro-5,6,8,9-tetrahydro-7*H*-5,9-propanobenzo[7]annulene-7,11-dione was obtained (8.73 g, 94% yield).
mp > 150 °C (dec.). IR (NaCl disk): 2940, 2908, 1701, 1619, 1585, 1468, 1421, 1370, 1303, 1245, 1222, 1203, 1072, 1052, 988, 931, 897, 789, 746 cm⁻¹. HRMS-ESI+ m/z [M+H]⁺ calcd for [C₁₄H₁₃FO₂+H]⁺: 233.0972, found: 233.0976.

### Reference example 5: 2-fluoro-7,11-dimethylene-6,7,8,9-tetrahydro-5H-5,9-propanobenzo[7]annulene.

In a 3-necked round-bottomed flask equipped with magnetic stirring and argon atmosphere, a suspension of NaH (1.08 g, 60 % purity, 27.0 mmol) in anhydrous DMSO (13.3 mL) was heated at 75 °C over 45 min. The green suspension was cooled down to room temperature and methyltriphenylphosphonium iodide (10.92 g, 27.0 mmol) diluted in anhydrous DMSO (22 mL) and 2-fluoro-5,6,8,9-tetrahydro-7*H*-5,9-propanobenzo[7]annulene-7,11-dione (1.53 g, 6.59 mmol) diluted in anhydrous DMSO (50 mL) were sequentially added. The resulting mixture was heated at 90 °C overnight. The reaction was cooled down and poured into water (80 mL). The aqueous layer was extracted with hexane (4 x 80 mL). The combined organic extracts were dried over anh. Na₂SO₄ filtered and concentrated under vacuum. Column chromatography (SiO₂, Hexane/Ethyl Acetate mixtures) gave 2-fluoro-7,11-dimethylene-6,7,8,9-tetrahydro-5*H*-5,9-propanobenzo[7]annulene as a colorless wax (1.09, 73% yield).
mp 108-109 °C. IR (NaCl disk): 3072, 2985, 2921, 2844, 1639, 1612, 1592, 1494, 1451, 1444, 1363, 1246, 1162, 1135, 1095, 1048, 974, 951, 930, 887, 820, 716, 658, 638, 598, 528 cm⁻¹.

### Reference example 6: 2-methoxy-7,11-dimethylene-6,7,8,9-tetrahydro-5H-5,9-propanobenzo[7]annulene.

From 2-methoxy-5,6,8,9-tetrahydro-7*H*-5,9-propanobenzo[7]annulene-7,11-dione (4 g, 16.4 mmol) and following the procedure described in reference example 5, 2-methoxy-7,11-dimethylene-6,7,8,9-tetrahydro-5*H*-5,9-propanobenzo[7]annulene was obtained (1.5 g, 38 % yield).
mp 68-69°C. IR (NaCl disk): 3068, 2979, 2911, 2833, 1639, 1609, 1580, 1501, 1464, 1449, 1431, 1363, 1313, 1260, 1203, 1172, 1152, 1109, 1034, 955, 929, 889, 851, 809, 661, 613 cm⁻¹. HRMS-ESI+ m/z [M+H]⁺ calcd for [C₁₇H₂₀O+H]⁺: 241.1587, found: 241.1588.

### Reference example 7: 2,3-dimethoxy-7,11-dimethylene-6,7,8,9-tetrahydro-5H-5,9-propanobenzo[7]annulene.

From 2,3-dimethoxy-5,6,8,9-tetrahydro-7*H*-5,9-propanobenzo[7]annulene-7,11-dione (2.8 g, 10.2 mmol) and following the procedure described in reference example 5, 2,3-dimethoxy-7,11-dimethylene-6,7,8,9-tetrahydro-5*H*-5,9-propanobenzo[7]annulene was obtained (633 mg, 23% yield).
mp 74-75 °C. IR (NaCl disk): 3068, 2977, 2913, 2832, 1639, 1606, 1515, 1464, 1450, 1429, 1414, 1358, 1342, 1293, 1261, 1240, 1225, 1191, 1173, 1103, 1023, 956, 931, 889, 804, 634 cm⁻¹. HRMS-ESI+ m/z [M+H]⁺ calcd for [C₁₈H₂₂O₂+H]⁺: 271.1693, found: 271.1688.

### Reference example 8: 1-fluoro-7,11-dimethylene-6,7,8,9-tetrahydro-5H-5,9-propanobenzo[7]annulene.

From 1-fluoro-5,6,8,9-tetrahydro-7*H*-5,9-propanobenzo[7]annulene-7,11-dione (4 g, 17.2 mmol) and following the procedure described in reference example 5, 1-fluoro-7,11-dimethylene-6,7,8,9-tetrahydro-5*H*-5,9-propanobenzo[7]annulene was obtained as a colourless oil (2.69 g, 69% yield).
IR (NaCl disk): 3071, 2981, 2921, 2838, 1639, 1614, 1583, 1464, 1446, 1429, 1365, 1248, 1046, 991, 935, 919, 895 cm⁻¹. HRMS-ESI+ m/z [M+H]⁺ calcd for [C₁₆H₁₇F+H]⁺: 229.1387, found: 229.1392.

### Reference example 9: 2-chloro-N-(2-fluoro-9-methyl-5,6,8,9,10,11-hexahydro-7H-5,9:7,11-dimethanobenzo[9]annulen-7-yl)acetamide.

A suspension of 2-fluoro-7,11-dimethylene-6,7,8,9-tetrahydro-5*H*-5,9-propanobenzo[7]annulene (1.09 g, 4.77 mmol), chloroacetonitrile (1.2 mL, 19.1 mmol) and acetic acid (3.5 mL) was cooled to 0 °C and concentrated H₂SO₄ (1.53 mL, 28.6 mmol) was added dropwise (T < 10 °C). The mixture was allowed to reach room temperature and was stirred overnight. The suspension was added to ice (20 g) and after 10 min stirring, the suspension was extracted with DCM (3 x 15 mL). The combined organic layers were washed with NaOH 10 N (1 x 25 mL) and dried with anh. Na₂SO₄ filtered and concentrated under vacuum to obtain 2-chloro-*N*-(2-fluoro-9-methyl-5,6,8,9,10,11-hexahydro-7*H*-5,9:7,11-dimethanobenzo[9]annulen-7-yl)acetamide as a white solid (1.2 g, 78% yield).
mp 141-144 °C. IR (NaCl disk): 3399, 3313, 3067, 2944, 2920, 2851, 1657, 1607, 1591, 1518, 1498, 1451, 1361, 1345, 1252, 1179, 1145, 966, 963, 863, 820 cm⁻¹. HRMS-ESI+ *m*/*z* [M+H]⁺ calcd for [C₁₈H₂₁ClFNO+H]⁺: 322.1368, found: 322.1370.

### Reference example 10: 2-chloro-N-(2-methoxy-9-methyl-5,6,8,9,10,11-hexahydro-7H-5,9:7,11-dimethanobenzo[9]annulen-7-yl)acetamide.

From 2-methoxy-7,11-dimethylene-6,7,8,9-tetrahydro-5*H*-5,9-propanobenzo[7] annulene (1.5 g, 6.24 mmol), and following the procedure described in reference example 9, 2-chloro-*N*-(2-methoxy-9-methyl-5,6,8,9,10,11-hexahydro-7*H*-5,9:7,11-dimethanobenzo[9]annulen-7-yl)acetamide was obtained (1.18 g, 57% yield).
mp 144-145 °C. IR (NaCl disk): 3403, 3304, 3062, 2997, 2945, 2905, 2860, 2838, 1662, 1609, 1582, 1528, 1499, 1454, 1382, 1361, 1311, 1267, 1242, 1198, 1180, 1154, 1043, 1013, 955, 873 cm⁻¹. HRMS-ESI+ m/z [M+H]⁺ calcd for [C₁₉H₂₄ClNO₂+H]⁺: 334.1568, found: 334.1569.

### Reference example 11: 2-chloro-N-(2,3-dimethoxy-9-methyl-5,6,8,9,10,11-hexahydro-7H-5,9:7,11-dimethanobenzo[9]annulen-7-yl)acetamide.

From 2,3-dimethoxy-7,11-dimethylene-6,7,8,9-tetrahydro-5*H*-5,9-propanobenzo[7] annulene (498 mg, 1.84 mmol), and following the procedure described in reference example 9, 2-chloro-*N*-(2,3-dimethoxy-9-methyl-5,6,8,9,10,11-hexahydro-7*H*-5,9:7,11-dimethanobenzo[9]annulen-7-yl)acetamide was obtained (501 mg, 75% yield).
mp 204-205 °C. IR (NaCl disk): 3306, 2941, 2907, 2861, 2838, 1666, 1605, 1516, 1467, 1452, 1415, 1381, 1361, 1345, 1293, 1252, 1231, 1191, 1168, 1092, 1021, 948, 861, 802 cm⁻¹. HRMS-ESI+ m/z [M+H]⁺ calcd for [C₂₀H₂₆ClNO₃+H]⁺: 364.1674, found: 364.1674.

### Reference example 12: 2-chloro-N-(1-fluoro-9-methyl-5,6,8,9,10,11-hexahydro-7H-5,9:7,11-dimethanobenzo[9]annulen-7-yl)acetamide.

From 1-fluoro-7,11-dimethylene-6,7,8,9-tetrahydro-5*H*-5,9-propanobenzo[7]annulene (2.36 g, 10.36 mmol), and following the procedure described in reference example 9, 2-chloro-*N*-(1-fluoro-9-methyl-5,6,8,9,10,11-hexahydro-7H-5,9:7,11-dimethanobenzo[9]annulen-7-yl)acetamide was obtained (2.28 g, 68% yield). The analytical sample was obtained by crystallization from DCM.
mp 154-155 °C. IR (NaCl disk): 3402, 3308, 3073, 2947, 2911, 2863, 2840, 1660, 1613, 1583, 1529, 1463, 1363, 1348, 1312, 1242, 1186, 1155, 979, 798, 748 cm⁻¹. HRMS-ESI+ m/z [M+H]⁺ calcd for [C₁₈H₂₁ClFNO+H]⁺: 322.1368, found: 322.1374.

### Reference example 13: 2-fluoro-9-methyl-5,6,8,9,10,11-hexahydro-7H-5,9:7,11-dimethanobenzo[9]annulen-7-amine hydrochloride.

Thiourea (25 mg, 0.32 mmol) and glacial acetic acid (200 µL) were added to a solution of 2-chloro-*N*-(2-fluoro-9-methyl-5,6,8,9,10,11-hexahydro-7*H*-5,9:7,11-dimethanobenzo [9]annulen-7-yl)acetamide (87 mg, 0.27 mmol) in absolute ethanol (5 mL) and the mixture was heated at reflux overnight. The resulting suspension was then tempered to room temperature, water (5 mL) was added and the pH adjusted to 12 with 5 N NaOH solution. EtOAc (5 mL) was added, the phases were separated and the aqueous phase was extracted with further EtOAc (2 x 5 mL). The combined organic layers were dried over anh. Na₂SO₄ filtered and concentrated under vacuum to give a light brown oil. Its hydrochloride was obtained by adding an excess of Et₂O/HCl to a solution of the amine in ethyl acetate, followed by filtration of the resulting beige precipitate affording 2-fluoro-9-methyl-5,6,8,9,10,11-hexahydro-7*H*-5,9:7,11 dimethanobenzo[9]annulen-7-amine hydrochloride (18 mg, 24% yield).
mp >300 °C (dec.). IR (KBr disk): 3200-2500 (2983, 2945, 2917, 2867), 2059, 1612, 1595, 1501, 1456, 1444, 1431, 1379, 1364, 1302, 1283, 1256, 1246, 1186, 1157, 1143, 1132, 1030, 1004, 962, 863, 814 cm⁻¹. Anal. Calcd for C₁₆H₂₀FN· 2.6 HCl: C 56.50, H 6.70, N 4.12. Found: C 56.18, H 6.40, N 4.01.

### Reference example 14: 2-methoxy-9-methyl-5,6,8,9,10,11-hexahydro-7H-5,9:7,11-dimethanobenzo[9]annulen-7-amine hydrochloride.

From 2-chloro-*N*-(2-methoxy-9-methyl-5,6,8,9,10,11-hexahydro-7*H*-5,9:7,11-dimethanobenzo[9]annulen-7-yl)acetamide (1.10 g, 3.95 mmol), and following the procedure described in reference example 13, 2-methoxy-9-methyl-5,6,8,9,10,11-hexahydro-7*H*-5,9:7,11-dimethanobenzo[9]annulen-7-amine hydrochloride was obtained. The analytical sample was obtained by crystallization from DCM/Pentane (779 mg, 81 % yield).
mp >250 °C (dec.). IR (KBr disk): 3200-2500 (2985, 2942, 2908), 2056, 1735, 1609, 1582, 1499, 1449, 1379, 1364, 1334, 1305, 1268, 1252, 1205, 1170, 1132, 1103, 1040, 1001, 954, 869, 849, 815, 756, 692 cm⁻¹. HRMS-ESI+ m/z [M+H]⁺ calcd for [C₁₇H₂₃NO+H]⁺: 258.1852, found: 258.1862.

### Reference example 15: 2,3-dimethoxy-9-methyl-5,6,8,9,10,11-hexahydro-7H-5,9:7,11-dimethanobenzo[9]annulen-7-amine hydrochloride.

From 2-chloro-*N*-(2,3-dimethoxy-9-methyl-5,6,8,9,10,11-hexahydro-7*H*-5,9:7,11-dimethanobenzo[9]annulen-7-yl)acetamide (436 mg, 1.2 mmol), and following the procedure described in reference example 13, 2,3-dimethoxy-9-methyl-5,6,8,9,10,11-hexahydro-7*H*-5,9:7,11-dimethanobenzo[9]annulen-7-amine hydrochloride was obtained (285 mg, 73% yield).
mp >200 °C (dec.). IR (KBr disk): 3200-2500 (2993, 2918, 2831), 2047, 1701, 1606, 1517, 1451, 1416, 1386, 1365, 1327, 1310, 1291, 1252, 1237, 1192, 1174, 1131, 1098, 1031, 973, 950, 863, 798, 586, 543 cm⁻¹. HRMS-ESI+ m/z [M+H]⁺ calcd for [C₁₈H₂₅NO₂+H]⁺: 288.1958, found: 288.1954.

### Reference example 16: 1-fluoro-9-methyl-5,6,8,9,10,11-hexahydro-7H-5,9:7,11-dimethanobenzo[9]annulen-7-amine hydrochloride.

From 2-chloro-*N*-(1-fluoro-9-methyl-5,6,8,9,10,11-hexahydro-7*H*-5,9:7,11-dimethanobenzo[9]annulen-7-yl)acetamide (1 g, 3.11 mmol), and following the procedure described in reference example 13, 1-fluoro-9-methyl-5,6,8,9,10,11-hexahydro-7H-5,9:7,11-dimethanobenzo[9]annulen-7-amine hydrochloride was obtained. The analytical sample was obtained by crystallization from Methanol (521 mg, 59 % yield).
mp >200 °C (dec.). IR (KBr disk): 3200-2500 (2945, 2717), 2060, 1677, 1608, 1584, 1511, 1464, 1390, 1380, 1366, 1317, 1303, 1248, 1214, 1199, 1165, 1132, 1071, 1052, 1032, 1000, 977, 946, 885, 877, 854, 798, 747, 623 cm⁻¹. HRMS-ESI+ m/z [M+H]⁺ calcd for [C₁₆H₂₀FN+H]⁺: 246.1653, found: 246.1649.

### Reference example 17: 2-chloro-N-(9-methyl-2-nitro-5,6,8,9,10,11-hexahydro-7H-5,9:7,11-dimethanobenzo[9]annulen-7-yl)acetamide.

To a cold (0°C) solution of known 2-chloro-*N*-(9-methyl-5,6,8,9,10,11-hexahydro-7*H-*5,9:7,11-dimethanobenzo[9]annulen-7-yl)acetamide (Bioorg Med Chem. 2012, 20, 942) (3 g, 9.87 mmol) in acetic anhydride (10.5 mL) were carefully added glacial acetic acid (1.6 mL) and fuming nitric acid (1.85 mL). The mixture was allowed to reach room temperature and left stirring overnight. The obtained yellow solution was then poured to ice-water (20 mL), and extracted with DCM (3 x 40 mL). The combined organic extracts were washed with aqueous 2 N NaOH (1 x 40 mL), water (1 x 40 mL) and brine (1 x 40 mL). The organic layer was dried over anh. Na₂SO₄ filtered and concentrated under vacuum to give a yellow residue. Purification by column chromatography (SiO₂, Hexane/Ethyl Acetate mixtures) gave 2-chloro-*N*-(9-methyl-2-nitro-5,6,8,9,10,11-hexahydro-7*H*-5,9:7,11-dimethanobenzo[9]annulen-7-yl)acetamide (2.92 g, 85% yield) as a white solid.
mp 174 °C. IR (KBr disk): 3403, 3288, 3077, 2946, 2922, 2847, 1667, 1607, 1588, 1520, 1456, 1409, 1348, 1229, 1166, 1136, 1082, 1051, 1009, 972, 945, 896, 865, 841, 797, 740, 764, 704, 666 cm⁻¹. HRMS-ESI+ m/z [M+H]⁺ calcd for [C₁₈H₂₁ClN₂O₃+H]⁺: 349.1313, found: 349.1313.

### Reference example 18: 9-methyl-2-nitro-5,6,8,9,10,11-hexahydro-7H-5,9:7,11-dimethanobenzo[9]annulen-7-amine hydrochloride.

From 2-chloro-*N*-(9-methyl-2-nitro-5,6,8,9,10,11-hexahydro-7*H*-5,9:7,11-dimethanobenzo[9]annulen-7-yl)acetamide (677 mg, 1.94 mmol) and following the procedure described in reference example 13, 9-methyl-2-nitro-5,6,8,9,10,11-hexahydro-7*H*-5,9:7,11-dimethanobenzo[9]annulen-7-amine hydrochloride was obtained (443 mg, 74% yield).
mp >225 °C (dec.). IR (KBr disk): 3200-2500 (2928, 2641, 2603, 2535), 2066, 1648, 1612, 1591, 1522, 1487, 1458, 1352, 1304, 1286, 1256, 1221, 1181, 1134, 1088, 1031, 955, 946, 895, 884, 865, 836, 799, 766 cm⁻¹. HRMS-ESI+ m/z [M+H]⁺ calcd for [C₁₆H₂₀N₂O₂+H]⁺: 273.1598, found: 273.1604.

### Reference example 19: 9-methyl-5,6,8,9,10,11-hexahydro-7H-5,9:7,11-dimethanobenzo[9]annulene-2,7-diamine dihydrochloride.

To a solution of amine 9-methyl-2-nitro-5,6,8,9,10,11-hexahydro-7*H*-5,9:7,11-dimethanobenzo[9]annulen-7-amine (201 mg, 0.738 mmol) in methanol (25 mL), Pd on charcoal (68.6 mg, cat. 10% Pd) was added and the resulting suspension was hydrogenated at 1 atm of H₂ at room temperature for 48 h. The black suspension was filtered and the solvent removed by concentration under vacuum to give 9-methyl-5,6,8,9,10,11-hexahydro-7*H*-5,9:7,11-dimethanobenzo[9]annulene-2,7-diamine as a brown solid (130 mg, 89% yield). Its dihydrochloride was obtained by addition of an excess of Et₂O/HCl to a solution of the diamine in methanol followed by filtration of the resulting brown precipitate.
mp 294-295 °C. IR (KBr disk): 3200-2500 (3024, 2912, 2847, 2588), 1994, 1598, 1502, 1454, 1381, 1365, 1303, 1261, 1173, 1131, 1021, 957, 877, 827, 576, 473 cm⁻¹. Anal. Calcd. for C₁₆H₂₂N₂·3.4 HCl: C 52.46, H 6.99, N 7.65. Found C 52.64, H 7.18, N 7.43.

### Reference example 20: 2-chloro-N-(2-hydroxy-9-methyl-5,6,8,9,10,11-hexahydro-7H-5,9:7,11-dimethanobenzo[9]annulen-7-yl)acetamide.

To a solution of *N*-(2-amino-9-methyl-5,6,8,9,10,11-hexahydro-7*H*-5,9:7,11-dimethanobenzo[9]annulen-7-yl)-2-chloroacetamide (999 mg, 3.10 mmol) in H₂O (5 mL) and conc. HCl (5 mL), at 0 °C, was added dropwise a solution of sodium nitrite (427 mg, 6.21 mmol) in H₂O (2 mL). To the resulting solution was added CuCl (652 mg, 6.56 mmol) in conc. HCI (3 mL) and over 10 min gas evolution was observed. The resulting solution was warmed to 60 °C for 90 minutes, then was cooled to room temperature, diluted in H₂O (60 mL) and extracted with DCM (4 x 90 mL). The combined organic extracts were washed with sat. NaHCO₃ and brine, were dried over anh. Na₂SO₄ filtered and concentrated under vacuum to give a dark green solid. Purification by column chromatography (SiO₂, Hexane/Ethyl Acetate mixture) gave the 2-chloro-*N*-(2-hydroxy-9-methyl-5,6,8,9,10,11-hexahydro-7*H*-5,9:7,11-dimethanobenzo[9]annulen-7-yl)acetamide (78 mg, 9% yield) as a white solid.
mp 98-100 °C. IR (NaCl disk): 3300-2700 (3266, 3186, 3118, 2966, 2942, 2916, 2861), 2175, 1590, 1568, 1504, 1451, 1426, 1376, 1356, 1309, 1267, 1161, 1130, 1081, 1058, 827, 804 cm⁻¹. HRMS-ESI- m/z [M-H]⁻ calcd for for [C₁₈H₂₂ClNO₂-H]⁻: 318.1266, found: 318.1272.

### Reference example 21: 2-hydroxy-9-methyl-5,6,8,9,10,11-hexahydro-7H-5,9:7,11-dimethanobenzo[9]annulen-7-amine hydrochloride.

From 2-chloro-*N*-(2-hydroxy-9-methyl-5,6,8,9,10,11-hexahydro-7*H*-5,9:7,11-dimethanobenzo[9]annulen-7-yl)acetamide (72 mg, 0.23 mmol), and following the procedure described in reference example 13, 2-hydroxy-9-methyl-5,6,8,9,10,11-hexahydro-7*H*-5,9:7,11-dimethanobenzo[9]annulen-7-amine hydrochloride was obtained (42 mg, 67% yield). The analytical sample was obtained by crystallization from Methanol/Diethyl ether.
mp 183-185 °C. Anal. Calcd for C₁₆H₂NO·1.7HCl·1H₂O: C 59.43, H 7.70, N 4.33. Found C 59.63, H 7.44, N 4.77.

### Reference example 22: N-(9-methyl-2-nitro-5,6,8,9,10,11-hexahydro-7H-5,9:7,11-dimethanobenzo[9]annulen-7-yl)acetamideyl)acetamide.

To a cold (0 °C) solution of known (Tetrahedron Lett. 1987, 28, 1585-1588) *N*-(9-methyl-5,6,8,9,10,11-hexahydro-7*H*-5,9:7,11-dimethanobenzo[9]annulen-7-yl)acetamide (2.68 g, 9.93 mmol) in acetic anhydride (10.6 mL) were carefully added glacial acetic acid (1.6 mL) and fuming nitric acid (1.86 mL). The mixture was allowed to reach room temperature and left stirring overnight. The obtained yellow solution was then poured to ice-water (20 mL), and extracted with DCM (3 x 40 mL). The combined organic extracts were washed with aqueous 2 N NaOH (1 x 40 mL), water (1 x 40 mL) and brine (1 x 40 mL). The organic layer was dried over anh. Na₂SO₄ filtered and concentrated under vacuum to give a yellow residue. Purification by column chromatography (SiO₂, Hexane/Ethyl Acetate mixtures) gave *N*-(9-methyl-2-nitro-5,6,8,9,10,11-hexahydro-7*H*-5,9:7,11-dimethanobenzo[9]annulen-7-yl)acetamide (1.88 g, 60% yield) as a white solid.
mp 174-176 °C. IR (NaCl disk): 3398, 3301, 3201, 3063, 2943, 2917, 2863, 1653, 1588, 1523, 1455, 1346, 1322, 1304, 1268, 1245, 1217, 1166, 1141, 1124, 1081, 1037, 1010, 945, 893, 865, 838, 798, 763, 740, 701 cm⁻¹. HRMS-ESI+ m/z [M+H]⁺ calcd for [C₁₈H₂₂N₂O₃+H]⁺: 315.1703, found: 315.1714.

### Reference example 23: N-(2-amino-9-methyl-5,6,8,9,10,11-hexahydro-7H-5,9:7,11-dimethanobenzo[9]annulen-7-yl)acetamide.

From *N*-(9-methyl-2-nitro-5,6,8,9,10,11-hexahydro-7*H*-5,9:7,11-dimethanobenzo[9]annulen-7-yl)acetamide (2.64 g, 8.41 mmol), PtO₂ (258 mg) in absolute EtOH and following the procedure described in the reference example 19, *N-*(2-amino-9-methyl-5,6,8,9,10,11-hexahydro-7*H*-5,9:7,11-dimethanobenzo[9]annulen-7-yl)acetamide (1.9 g, 80% yield) was obtained after purification by column chromatography (SiO₂, Hexane/Ethyl Acetate mixtures).
mp 112-113 °C. IR (NaCl disk): 3432, 3324, 3224, 3056, 3004, 2938, 2903, 2856, 2835, 1651, 1618, 1546, 1507, 1447, 1362, 1344, 1300, 1262, 1194, 1164, 1136, 1065, 862, 735, 701 cm⁻¹. HRMS-ESI+ m/z [M+H]⁺ calcd for [C₁₈H₂₄N₂O+H]⁺: 285.1961, found: 285.1972.

### Reference example 24: N-(2-chloro-9-methyl-5,6,8,9,10,11-hexahydro-7H-5,9:7,11-dimethanobenzo[9]annulen-7-yl)acetamide.

From *N*-(2-amino-9-methyl-5,6,8,9,10,11-hexahydro-7*H*-5,9:7,11-dimethanobenzo[9] annulen-7-yl)acetamide hydrochloride (1.04 g, 3.25 mmol) in H₂O (6 mL) and conc. HCl (6 mL), sodium nitrite (448 mg, 6.5 mmol) in H₂O (2 mL), CuCI (691 mg, 6.99 mmol) dissolved in conc. HCl solution (3 mL), and following the procedure described in reference example 20, *N*-(2-chloro-9-methyl-5,6,8,9,10,11-hexahydro-7*H*-5,9:7,11-dimethanobenzo[9]annulen-7-yl)acetamide was obtained (210 mg, 21% yield).
mp 190-191 °C. IR (NaCl disk): 3301, 3196, 3071, 2921, 2855, 1651, 1594, 1549, 1487, 1454, 1414, 1364, 1343, 1308, 1281, 1263, 1211, 1139, 1109, 1012, 950, 875, 820 cm⁻¹. HRMS-ESI+ m/z [M+H]⁺ calcd for [C₁₈H₂₂ClNO+H]⁺: 304.1463, found: 304.1460.

### Reference example 25: 2-chloro-9-methyl-5,6,8,9,10,11-hexahydro-7H-5,9:7,11-dimethanobenzo[9]annulen-7-amine hydrochloride.

A mixture of *N*-(2-chloro-9-methyl-5,6,8,9,10,11-hexahydro-7*H*-5,9:7,11-dimethanobenzo[9]annulen-7-yl)acetamide (190 mg, 0.63 mmol), conc. HCl (4 mL), H₂O (8 mL) and isopropanol (6 mL) was stirred under reflux for 4 days. The solution was cooled down and isopropanol was concentrated under vacuum. The aqueous phase was extracted with EtOAc (3 x 8 mL) and then was basified with a solution of 5 N NaOH. The base aqueous solution was extracted with further EtOAc (3 x 10 mL), dried over anh. Na₂SO₄ filtered and concentrated under vacuum to give a yellow oil. Purification by column chromatography (SiO₂, Hexane/Ethyl Acetate mixtures) gave 2-chloro-9-methyl-5,6,8,9,10,11-hexahydro-7*H*-5,9:7,11-dimethanobenzo[9]annulen-7-amine. Its hydrochloride was obtained by adding an excess of Et₂O/HCl to a solution of the amine in EtOAc (10 mg, 5.5% yield).
mp > 250 °C. IR (KBr disk): 3200-2500 (2990, 2950, 2916, 2861), 2058, 1597, 1570, 1509, 1488, 1454, 1416, 1380, 1365, 1302, 1217, 1155, 1133, 1093, 1032, 1000, 948, 875, 820, 771, 673 cm⁻¹. Anal. Calcd for C₁₆H₂₀ClN·1.35 HCl: C 61.79, H 6.95, N 4.50. Found C 61.70, H 6.78, N 4.93.

### Reference example 26: 5,8,9,10-tetrahydro-5,8:7,10-dimethanobenzo[8]annulen-7(6H)-ylmethanesulfonate.

To a solution of 5,8,9,10-tetrahydro-5,8:7,10-dimethanobenzo[8]annulen-7(6*H*)-ol (1.19 g, 5.97 mmol) in pyridine (9 mL), (prepared as reported in Liebigs Ann Chem. 1973; 1839-1850), mesyl chloride (2.32 mL, 29.28 mmol) was added slowly with stirring at room temperature. The mixture was then heated at 120 °C for 5 h. After cooling, crushed ice (100 g) was added and the mixture was extracted with DCM (5 x 40 mL). The combined organic phase was washed with 2 N HCl (2 x 40 mL), H₂O (2 x 40 mL), saturated aqueous NaHCO₃ (2 x 40 mL), and dried over anh. Na₂SO₄. After filtration and removal of the solvent under reduced pressure, 5,8,9,10-tetrahydro-5,8:7,10-dimethanobenzo[8]annulen-7(6*H*)-yl methanesulfonate (1.32 g, 80% yield) was isolated as a dark oil that was used in the next step without further purification.
IR (NaCl disk): 3060, 3010, 2934, 2857, 1488, 1451, 1341, 1232, 1175, 1145, 1102, 1046, 1012, 992, 966, 923, 853, 800, 753 cm⁻¹. HRMS-ESI+ m/z [M+H]⁺ calcd for [C₁₅H₁₈O₃S+NH₄]⁺: 296.1315, found: 296.1318.

### Reference example 27: 7-iodo-5,6,7,8,9,10-hexahydro-5,8:7,10-dimethanobenzo[8] annulene.

A mixture of H₃PO₄ (99%, 135 g), 5,8,9,10-tetrahydro-5,8:7,10-dimethanobenzo[8]annulen-7(6*H*)-yl methanesulfonate (1.32 g, 4.75 mmol) and NaI (63 g, 420 mmol) were stirred at 150 °C for 6 h. After cooling, H₂O (150 mL) was added slowly to the mixture. The resulting purple solution was extracted with DCM (4 x 80 mL) and the combined organic phase was washed with 10% aqueous sodium thiosulfate (1 x 100 mL), dried over anh. Na₂SO₄ and the solvent was removed under vacuum to obtain 7-iodo-5,6,7,8,9,10-hexahydro-5,8:7,10-dimethanobenzo[8]annulene as a white solid (1.39 g, 95 %).
mp 132-133 °C. IR (NaCl disk) 3052, 3013, 2950, 2892, 2852, 1490, 1447, 1304, 1278, 1232, 1215, 1095, 1046, 1032, 967, 830, 778, 755 cm⁻¹. GC-MS (El): 310 [(M)˙⁺, 2], 183 [(M-I)⁺, 100], 141 (73), 129 (23), 128 (15).

### Reference example 28: 5,8,9,10-tetrahydro-5,8:7,10-dimethanobenzo[8]annulene-7(6H)-carboxylic acid.

To a solution of 7-iodo-5,8,9,10-tetrahydro-5,8:7,10-dimethanobenzo[8]annulene (2.03 g, 6.5 mmol) in dry and degassed toluene (20 mL) was added methyl oxalyl chloride (2.39 g, 19.5 mmol) and bis(tributyltin) (4.5 g, 7.8 mmol). The mixture was irradiated in a quartz reactor under argon atmosphere with a 125 W Hg lamp for 20 h. Then, DCM (15 mL), methanol (0.6 mL) and triethylamine (1.2 mL) were successively added to the reaction mixture at 0° C and was concentrated under vacuum to give a dark oil (3.99 g). A solution of this oil in a 40% methanol solution of KOH (50 mL) was heated to reflux for 2 h. Water (50 mL) was added and the reaction was refluxed for 3 h. The reaction mixture was allowed to cool down to room temperature and the methanol was removed under vacuum. Water (40 mL) was added to the residue and the aqueous layer was washed with DCM (4 x 50 mL). After that, the aqueous phase was acidified with conc. HCl until pH=1 and extracted with DCM (4 x 50 mL). The organic extracts were dried over anh. Na₂SO₄, filtered and concentrated under reduced pressure to give 5,8,9,10-tetrahydro-5,8:7,10-dimethanobenzo[8]annulene-7(6*H*)-carboxylic acid as a brown solid (555 mg, 37% overall yield). An analytical sample of the acid was obtained by crystallization from DCM/Pentane.
mp 188-189 °C. IR (NaCl disk): 3300-2800 (3065, 3011, 2946, 2858), 1690, 1488, 1450, 1410, 1318, 1290, 1231, 1218, 1092, 1052, 1038, 941 cm⁻¹. HRMS-ESI- m/z [M-H]⁻ calcd for [C₁₅H₁₆O₂-H]⁻: 227.1078, found: 227.1078.

### Reference example 29: 5,8,9,10-tetrahvdro-5,8:7,10-dimethanobenzo[8]annulen-7(6H)-amine hydrochloride.

To a solution of 5,8,9,10-tetrahydro-5,8:7,10-dimethanobenzo[8]annulene-7(6*H*)-carboxylic acid (90 mg, 0.39 mmol) in toluene (1.2 mL), Et₃N (73 µL, 0.53 mmol) and diphenylphosphoryl azide (159 mg, 0.58 mmol) were added and heated at reflux for 3 h. The mixture was cooled down and washed with 1 N HCl (10 X 2 mL). Thereafter, to the organic layer was added 6 N HCl (1.6 mL) and the suspension was heated at reflux for 24 h. The reaction mixture was then cooled to room temperature and the two phases were separated. The aqueous phase was extracted with ethyl acetate (3 x 3 mL). The combined organic phases were washed with 5 N NaOH (3 x 10 mL), dried over anh. Na₂SO₄, filtered and concentrated under vacuum to give 5,8,9,10-tetrahydro-5,8:7,10-dimethanobenzo[8]annulen-7(6*H*)-amine. Its hydrochloride was obtained by adding an excess of HCl in methanol to a solution of the amine in methanol. The methanol was removed under reduced pressure to give 5,8,9,10-tetrahydro-5,8:7,10-dimethanobenzo[8]annulen-7(6H)-amine hydrochloride as a brown solid (35 mg, 45% yield). An analytical sample was obtained by crystallization from Methanol/Diethyl ether.
mp > 250 °C (dec). IR (KBr disk): 3100-2500 (2943, 2881), 2043, 1622, 1598, 1501, 1448, 1336, 1246, 1089, 1057, 1028, 952, 769, 749, 614 cm⁻¹. HRMS-ESI+ m/z [M+H]⁺ calcd for [C₁₄H₁₇N+H]⁺: 200.1434, found: 200.1432.

### Example 30: p-tolyl (9-methyl-5,6,8,9,10,11-hexahydro-7H-5,9:7,11-dimethanobenzo[9]annulen-7-yl)acetamide-yl)carbamate.

To a solution of 9-methyl-5,6,8,9,10,11-hexahydro-7*H*-5,9:7,11-dimethanobenzo[9] annulen-7-amine hydrochloride (250 mg, 0.95 mmol) in DCM (2 mL), *p*-tolyl chloroformate (194 mg, 1.14 mmol) and Et₃N (287 mg, 2.84 mmol) were added. The reaction mixture was stirred at room temperature overnight and then the solvent was evaporated under vacuum. The residue was dissolved in EtOAc (30 mL) and water (20 mL) and phases were separated. The aqueous phase was extracted with further EtOAc (2 x 30 mL). The combined organic phases were dried over anh. Na₂SO₄ filtered and concentrated under vacuum to obtain 300 mg of a yellow gum. Column chromatography (SiO₂, Hexane/Ethyl Acetate mixtures) gave *p*-tolyl (9-methyl-5,6,8,9,10,11-hexahydro-7*H*-5,9:7,11-dimethanobenzo[9]annulen-7-yl)carbamate (46 mg, 14% yield) as a white solid.
mp 114-115 °C. IR (NaCl disk): 3330, 3018, 2944, 2919, 2854, 1744, 1591, 1531, 1502, 1452, 1379, 1362, 1345, 1255, 1214, 1198, 1167, 1137, 1069, 1042, 1014, 987, 948, 900, 825, 757 cm⁻¹. Anal. Calcd for C₂₄H₂₇NO₂ · 0.3 C₅H₁₂ · 0.05 CH₂Cl₂: C 79.22, H 7.99, N 3.62. Found: C 79.23, H 7.88, N 3.45.

### Example 31: 1-(9-methyl-5,6,8,9,10,11-hexahydro-7H-5,9:7,11-dimethanobenzo[9] annulen-7-yl)-3-(4-(trifluoromethyl)phenyl)thiourea.

To a solution of 9-methyl-5,6,8,9,10,11-hexahydro-7*H*-5,9:7,11-dimethanobenzo[9]annulen-7-amine hydrochloride (250 mg, 0.95 mmol) in DCM (2 mL), 1-isothiocyanato-4-(trifluoromethyl)benzene (193 mg, 0.95 mmol) and Et₃N (287 mg, 2.84 mmol) were added. The reaction mixture was stirred at room temperature overnight and then the solvent was evaporated under vacuum. The residue was dissolved in EtOAc (30 mL) and water (20 mL) and phases were separated. The aqueous phase was extracted with further EtOAc (2 x 30 mL). The combined organic phases were dried over anh. Na₂SO₄ filtered and concentrated under vacuum to obtain 369 mg of a yellow solid. The product was washed with Et₂O to obtain 1-(9-methyl-5,6,8,9,10, 11-hexahydro-7*H*-5,9:7, 11-dimethanobenzo[9]annulen-7-yl)-3-(4-(trifluoromethyl)phenyl)thiourea (188 mg, 46% yield) as a white solid.
mp 158-159°C. IR (NaCl disk): 3283, 2911, 2834, 1615, 1532, 1493, 1454, 1422, 1324, 120, 1166, 1124, 1067, 1015, 948, 909, 837, 759, 732, 697, 665 cm⁻¹. Anal. Calcd for C₂₄H₂₅F₃N₂S: C 66.96, H 5.85, N 6.51. Found: C 66.79, H 5.95, N 6.37.

### Example 32: 1-(1-acetylpiperidin-4-yl)-3-(5-methyl-1,5,6,7-tetrahydro-1,5:3,7-dimethanobenzo[e]oxonin-3(2H)-yl)urea.

To a solution of 5-methyl-1,5,6,7-tetrahydro-1,5:3,7-dimethanobenzo[*e*]oxonin-3(2*H*)-amine hydrochloride (180 mg, 0.69 mmol) in DCM (3 mL) and saturated aqueous NaHCO₃ solution (2 mL), triphosgene (102 mg, 0.34 mmol) was added. The biphasic mixture was stirred at room temperature for 30 minutes and then the two phases were separated and the organic one was washed with brine (5 mL), dried over anh. Na₂SO₄, filtered and evaporated under vacuum to obtain 1-2 mL of a solution of isocyanate in DCM. To this solution were added 1-(4-aminopiperidin-1-yl)ethan-1-one hydrochloride (122 mg, 0.68 mmol) and Et₃N (139 mg, 1.37 mmol). The mixture was stirred overnight at room temperature, diluted with further DCM (10 mL) and washed with 2N NaOH solution (2 x 10 mL). The organic layer was dried over anh. Na₂SO₄, filtered and concentrated under vacuum to obtain a yellow residue (206 mg). Column chromatography (SiO₂, DCM/Methanol mixtures) gave 1-(1-acetylpiperidin-4-yl)-3-(5-methyl-1,5,6,7-tetrahydro-1,5:3,7-dimethanobenzo[e]oxonin-3(2*H*)-yl)urea as a white solid (135 mg, 49% yield). The analytical sample was obtained by crystallization from hot EtOAc (112 mg).
mp 208-209 °C. IR (NaCl disk): 3357, 3054, 3012, 2969, 2926, 2853, 1646, 1611, 1546, 1492, 1450, 1358, 1324, 1268, 1222, 1156, 1101, 1088, 1035, 1212, 991, 947, 918, 900, 866, 829, 760, 733, 699 cm⁻¹. Anal. Calcd for C₂₃H₃₁N₃O₃: C 69.49, H 7.86, N 10.57. Found: C 69.47, H 7.92, N 10.38.

### Example 33: 1-(1-acetylpiperidin-4-yl)-3-(1,5,6,7-tetrahydro-1,5:3,7-dimethano-benzo[e]oxonin-3(2H)-yl)urea.

To a solution of 1,5,6,7-tetrahydro-1,5:3,7-dimethanobenzo[*e*]oxonin-3(2*H*)-amine hydrochloride (300 mg, 1.19 mmol) in DCM (7 mL) and saturated aqueous NaHCO₃ solution (7 mL), triphosgene (130 mg, 0.44 mmol) was added. The biphasic mixture was stirred at room temperature for 30 minutes and then the two phases were separated and the organic one was washed with brine (10 mL), dried over anh. Na₂SO₄, filtered and evaporated under vacuum to obtain 1-2 mL of a solution of isocyanate in DCM. To this solution were added 1-(4-aminopiperidin-1-yl)ethan-1-one hydrochloride (203 mg, 1.43 mmol) and Et₃N (292 mg, 2.88 mmol). The mixture was stirred overnight at room temperature, diluted with further DCM (10 mL) and washed with 2N NaOH solution (2 x 10 mL). The organic layer was dried over anh. Na₂SO₄, filtered and concentrated under vacuum to obtain a yellow residue (400 mg). Column chromatography (SiO₂, DCM/Methanol mixtures) gave 1-(1-acetylpiperidin-4-yl)-3-(1,5,6,7-tetrahydro-1,5:3,7-dimethanobenzo[*e*]oxonin-3(2*H*)-yl)urea as a white solid (50 mg, 49% yield).
mp 200-202 °C. IR (NaCl disk): 3347, 3065, 3016, 2922, 1645, 1624, 1548, 1492, 1451, 1436, 1362, 1323, 1268, 1230, 1211, 1196, 1109, 1073, 1022, 980, 967 cm⁻¹.HRMS-ESI+ m/z [M+H]⁺ calcd for [C₂₂H₂₉N₃O₃+H]⁺: 384.2282, found: 384.2285.

### Example 34: 1-(1-acetylpiperidin-4-yl)-3-(9-methyl-5,6,8,9,10,11-hexahydro-7H-5,9:7,11-dimethanobenzo[9]annulen-7-yl)urea.

To a solution of 5-methyl-1,5,6,7-tetrahydro-1,5:3,7-dimethanobenzo[*e*]oxonin-3(2*H*)-amine hydrochloride (180 mg, 0.69 mmol) in DCM (3 mL) and saturated aqueous NaHCO₃ solution (2 mL), triphosgene (102 mg, 0.34 mmol) was added. The biphasic mixture was stirred at room temperature for 30 minutes and then the two phases were separated and the organic layer was washed with brine (5 mL), dried over anh. Na₂SO₄, filtered and evaporated under vacuum to obtain 1-2 mL of a solution of the isocyanate in DCM. To this solution were added 1-(4-aminopiperidin-1-yl)ethan-1-one hydrochloride (122 mg, 0.68 mmol) and Et₃N (138 mg, 1.36 mmol). The mixture was stirred overnight at room temperature, diluted with further DCM (10 mL) and washed with 2N NaOH solution (2 x 10 mL). Organics were dried over anh. Na₂SO₄, filtered and concentrated under vacuum to obtain a yellow oil (232 mg). Column chromatography (SiO₂, DCM/Methanol mixtures) gave 1-(1-acetylpiperidin-4-yl)-3-(9-methyl-5,6,8,9, 10,11-hexahydro-7*H*-5,9:7,11-dimethanobenzo[9]annulen-7-yl)urea as a white solid (143 mg, 53% yield). The analytical sample was obtained by crystallization from hot EtOAc (113 mg).
mp 206-207 °C. IR (NaCl disk): 3359, 3065, 3016, 2938, 2906, 2860, 1644, 1620, 1555, 1493, 1452, 1360, 1344, 1319, 1267, 1228, 1212, 1136, 1090, 1049 cm⁻¹. Anal. Calcd for C₂₄H₃₃N₃O₂·0.21 Ethyl Acetate: C 71.91, H 8.45, N 10.06. Found: C 71.73, H 8.43, N 10.27.

### Example 35: 1-(1-acetylpiperidin-4-yl)-3-(9-hydroxy-5,6,8,9,10,11-hexahydro-7H-5,9:7,11-dimethanobenzo[9]annulen-7-yl)urea.

To a solution of 1-(4-aminopiperidin-1-yl)ethan-1-one (192 mg, 1.35 mmol) in DCM (4 mL) and saturated aqueous NaHCO₃ solution (3 mL) triphosgene (200 mg, 0.68 mmol) was added. The biphasic mixture was stirred at room temperature for 30 minutes and then the two phases were separated and the organic one was washed with brine (5 mL), dried over anh. Na₂SO₄, filtered and evaporated under vacuum to obtain 1-2 mL of a solution of the isocyanate in DCM. To this solution was added 9-amino-5,6,8,9,10,11-hexahydro-7*H*-5,9:7,11-dimethanobenzo[9]annulen-7-ol hydrochloride (300 mg, 1.14 mmol) followed by Et₃N (228 mg, 2.25 mmol). The reaction mixture was stirred at room temperature overnight and the solvent was evaporated under vacuum. Column chromatography (SiO₂, DCM/Methanol mixtures) gave 1-(1-(isopropylsulfonyl)piperidin-4-yl)-3-(9-methyl-5,6,8,9,10,11-hexahydro-7*H*-5,9:7,11-dimethanobenzo[9]annulen-7-yl)urea (19 mg, 4.2% yield) as a grey solid.
mp 222-223 °C. IR (NaCl disk): 3313, 2921, 2852, 1733, 1716, 1646, 1621, 1557, 1542, 1506, 1490, 1472, 1455, 1358, 1336, 1318, 1300, 1265, 1231, 1204, 1134, 1104, 1053 cm⁻¹. Anal. Calcd for C₂₃H₃₁N₃O₃·0.2 C₅H₁₂ · 0.9 H₂O: C 67.33, H 8.29, N 9.81. Found: C 67.25, H 8.15, N 9.72

### Example 36: 1-(1-acetylpiperidin-4-yl)-3-(9-methoxy-5,6,8,9,10,11-hexahydro-7H-5,9:7,11-dimethanobenzo[9]annulen-7-yl)urea.

To a solution of 9-methoxy-5,6,8,9,10,11-hexahydro-7H-5,9:7,11-dimethano-benzo[9]annulen-7-amine (300 mg, 1.23 mmol) in DCM (4.5 mL) and saturated aqueous NaHCO₃ solution (3 mL) triphosgene (183 mg, 0.61 mmol) was added. The biphasic mixture was stirred at room temperature for 30 minutes and then the two phases were separated and organics were washed with brine (5 mL), dried over anh. Na₂SO₄ filtered and evaporated under vacuum to obtain 1-2 mL of a solution of the isocyanate in DCM. To this solution was added 1-(4-aminopiperidin-1-yl)ethan-1-one (210 mg, 1.47 mmol). The reaction mixture was stirred at room temperature overnight and the solvent was evaporated under vacuum to obtain a white gum (521 mg). Column chromatography (SiO₂, DCM/Methanol mixtures) gave 1-(1-acetylpiperidin-4-yl)-3-(9-methoxy-5,6,8,9,10,11-hexahydro-7*H*-5,9:7,11-dimethanobenzo[9]annulen-7-yl)urea (148 mg, 30% yield) as a white solid. The analytical sample was obtained by crystallization from hot EtOAc (119 mg).
mp 212-213 °C. IR (NaCl disk): 3358, 2930, 2847, 1646, 1617, 1555, 1495, 1451, 1356, 1319, 1266, 1228, 1094, 1075, 972, 849, 755, 735 cm⁻¹. Anal. Calcd for C₂₄H₃₃N₃O₃·0.15 EtOAc: C 69.56, H 8.12, N 9.89. Found: C 69.63, H 8.28, N 8.86

### Example 37: 1-(1-acetylpiperidin-4-yl)-3-(9-fluoro-5,6,8,9,10,11-hexahydro-7H-5,9:7,11-dimethanobenzo[9]annulen-7-yl)urea.

To a solution of 9-fluoro-5,6,8,9,10,11-hexahydro-7*H*-5,9:7,11-dimethanobenzo[9] annulen-7-amine (143 mg, 0.53 mmol) in DCM (4 mL) and saturated aqueous NaHCO₃ solution (2 mL) was added triphosgene (78 mg, 0.26 mmol). The biphasic mixture was stirred at room temperature for 30 minutes and then the two phases were separated and the organic layer was washed with brine (5 mL), dried over anh. Na₂SO₄, filtered and evaporated under vacuum to obtain 1-2 mL of a solution of the isocyanate in DCM. To this solution was added 1-(4-aminopiperidin-1-yl)ethan-1-one (90 mg, 0.63 mmol). The reaction mixture was stirred at room temperature overnight and the solvent was evaporated under vacuum to obtain a yellow gum (259 mg). Column chromatography (SiO₂, DCM/Methanol mixtures) gave 1-(1-acetylpiperidin-4-yl)-3-(9-fluoro-5,6,8,9,10,11-hexahydro-7H-5,9:7,11-dimethanobenzo[9]annulen-7-yl)urea (180 mg, 85% yield). The analytical sample was obtained by crystallization from hot DCM (57 mg).
mp 228-229 °C. IR (NaCl disk): 3357, 2927, 2856, 1643, 1618, 1553, 1494, 1451, 1358, 1340, 1316, 1267, 1227, 1207, 1134, 1097, 1042, 1004 cm⁻¹. Anal. Calcd for C₂₃H₃₆FN₃O₂·0.15 C₅H₁₂ · 0.62 H₂O: C 67.59, H 7.91, N 9.96. Found: C 67.61, H 7.93, N 8.94.

### Example 38: 1-(1-acetylpiperidin-4-yl)-3-(9-chloro-5,6,8,9,10,11-hexahydro-7H-5,9:7,11-dimethanobenzo[9]annulen-7-yl)urea.

To a solution of 9-chloro-5,6,8,9,10,11-hexahydro-7*H*-5,9:7,11-dimethanobenzo[9] annulen-7-amine hydrochloride (150 mg, 0.53 mmol) in DCM (3 mL) saturated aqueous NaHCO₃ solution (3 mL) and triphosgene (58 mg, 0.20 mmol) were added. The biphasic mixture was stirred at room temperature for 30 minutes and then the two phases were separated and the organic layer was washed with brine (3 mL), dried over anh. Na₂SO₄, filtered and concentrated under vacuum to obtain 1-2 mL of a solution of the isocyanate in DCM. To this solution was added 1-(4-aminopiperidin-1-yl)ethan-1-one (90 mg, 0.63 mmol). The reaction mixture was stirred at room temperature overnight and the solvent was evaporated under vacuum to obtain a white solid (204 mg). Column chromatography (SiO₂, DCM/Methanol mixtures) gave 1-(1-acetylpiperidin-4-yl)-3-(9-chloro-5,6,8,9,10,11-hexahydro-7*H*-5,9:7,11-dimethanobenzo[9]annulen-7-yl)urea (115 mg, 55% yield) as a white solid.
mp 209-210 °C. IR (NaCl disk): 3358, 3019, 2926, 2855, 1644, 1619, 1556, 1494, 1452, 1358, 1319, 1301, 1268, 1228, 1206, 1135, 1090, 1050, 991, 969, 947, 802, 761, 735 cm⁻¹. HRMS-ESI+ m/z [M+H]⁺ calcd for [C₂₃H₃₀ClN₃O₂+H]⁺: 416.2099, found: 416.2100.

### Example 39: 4-(((1r,4r)-4-(3-(5-methy)-1,5,6,7-tetrahydro-1,5:3,7-dimethanobenzo [e]oxonin-3(2H)-yl)ureido)cyclohexyl)oxy)benzoic acid.

To a solution of 5-methyl-1,5,6,7-tetrahydro-1,5:3,7-dimethanobenzo[*e*]oxonin-3(2*H*)-amine hydrochloride (200 mg, 0.76 mmol) in DCM (3.5 mL) and saturated aqueous NaHCO₃ solution (2.2 mL) was added triphosgene (113 mg, 0.38 mmol). The biphasic mixture was stirred at room temperature for 30 minutes and then the two phases were separated and the organic layer was washed with brine (5 mL), dried over anh. Na₂SO₄, filtered and evaporated under vacuum to obtain 1-2 mL of a solution of isocyanate in DCM. To this solution were added 4-(((1r,4r)-4-aminocyclohexyl)oxy)benzoic acid hydrochloride (206 mg, 0.76 mmol) and Et₃N (153 mg, 1.52 mmol). The mixture was stirred overnight at room temperature. The resulting suspension was evaporated to obtain a white solid, which was suspended in DCM (20 mL) and washed with 2N HCl solution (2 x 10 mL). The resulting organic suspension was filtered to afford a white solid (200 mg, 54% yield).
mp: 220-222 °C. IR (NaCl disk): 3352, 2626, 1678, 1601, 1558, 1506, 1454, 1373, 1343, 1312, 1288, 1247, 1221, 1161, 1104, 1029, 997, 953, 776 cm⁻¹. HRMS-ESI+ m/z [M+H]⁺ calcd for [C₂₉H₃₄N₂O₅+H]⁺: 491.254, found: 491.254.

### Example 40: 4-(((1r,4r)-4-(3-(9-methyl-5,6,8,9,10,11-hexahydro-7H-5,9:7,11-dimethanobenzo[9]annulen-7-yl)ureido)cyclohexyl)oxy)benzoic acid.

To a solution of 5-methyl-1,5,6,7-tetrahydro-1,5:3,7-dimethanobenzo[*e*]oxonin-3(2*H*)-amine hydrochloride (180 mg, 0.69 mmol) in DCM (3 mL) and saturated aqueous NaHCO₃ solution (2 mL) was added triphosgene (102 mg, 0.34 mmol). The biphasic mixture was stirred at room temperature for 30 minutes and then the two phases were separated and the organic layer was washed with brine (5 mL), dried over anh. Na₂SO₄ filtered and evaporated under vacuum to obtain 1-2 mL of a solution of isocyanate in DCM. To this solution were added 4-(((1r,4r)-4-aminocyclohexyl)oxy)benzoic acid hydrochloride (206 mg, 0.76 mmol) and Et₃N (153 mg, 1.52 mmol). The mixture was stirred overnight at room temperature. The resulting suspension was evaporated and the residue was suspended in DCM (20 mL) and washed with 2N HCl solution (2 x 10 mL). The resulting organic suspension was filtered and the filtrate was dried over anh. Na₂SO₄ filtered and concentrated under vacuum to give a white gum. Crystallization from hot EtOAc provided 4-(((1r,4r)-4-(3-(9-methyl-5,6,8,9,10, 11-hexahydro-7*H*-5,9:7, 11-dimethanobenzo[9]annulen-7-yl)ureido)cyclohexyl)oxy)benzoic acid as a white solid (55 mg, 16% yield).
mp 182-183 °C. IR (NaCl disk): 3335, 2921, 2855, 1692, 1681, 1642, 1632, 1602, 1564, 1537, 1504, 1494, 1469, 1453, 1419, 1360, 1307, 1248, 1163, 1122, 1096, 1969 cm⁻¹. Anal. Calcd for C₃₀H₃₆N₂O₄·1,5 H₂O: C 69.88, H 7.62, N 5.43. Found: C 69.53, H 7.37, N 5.10.

### Reference example 41: tert-butyl [1-(isopropylsulfonyl)piperidin-4-yl]carbamate.

To a solution of *tert*-butyl (piperidin-4-yl)carbamate (850 mg, 4.24 mmol) in DCM (7 mL) was added Et₃N (858 mg, 8.48 mmol). The mixture was cooled down to 0 °C with an ice bath and then propane-2-sulfonyl chloride (725 mg, 5.09 mmol) was added dropwise. The reaction mixture was stirred at room temperature overnight. The suspension was washed with 2N NaOH solution (2 x 5 mL) and the organic phase was dried over anh. Na₂SO₄ filtered and concentrated under vacuum to obtain *tert*-butyl [1-(isopropylsulfonyl)piperidin-4-yl] carbamate (1.15 g, 89% yield).

### Reference example 42: 1-(isopropylsulfonyl)piperidin-4-amine.

To a solution of *tert*-butyl (1-(isopropylsulfonyl)piperidin-4-yl)carbamate (1.15 g, 3.75 mmol) in dissolved in DCM (5 mL) and 4 M HCI in 1,4-dioxane (2 mL) was added. The mixture was stirred at room temperature for 2 days and the solvents were evaporated under vacuum. The residue was then dissolved in DCM (5 mL) and washed with 5N NaOH solution (5 mL). The organic layer was dried over anh. Na₂SO₄, filtered and concentrated under vacuum to give 1-(isopropylsulfonyl)piperidin-4-amine (704 mg, 91% yield) as a yellow oil.

### Example 43: 1-[1-(isopropylsulfonyl)piperidin-4-yl]-3-(9-methyl-5,6,8,9,10,11-hexahydro-7H-5,9:7,11-dimethanobenzo[9]annulen-7-yl)urea.

To a solution of 5-methyl-1,5,6,7-tetrahydro-1,5:3,7-dimethanobenzo[*e*]oxonin-3(2*H*)-amine hydrochloride (300 mg, 1.13 mmol) in DCM (6 mL) and saturated aqueous NaHCO₃ solution (4 mL) was added triphosgene (169 mg, 0.57 mmol). The biphasic mixture was stirred at room temperature for 30 minutes and then the two phases were separated and the organic layer was washed with brine (5 mL), dried over anh. Na₂SO₄, filtered and evaporated under vacuum to obtain 1-2 mL of a solution of the isocyanate in DCM.

To a solution of 1-(isopropylsulfonyl)piperidin-4-amine (233 mg, 1.13 mmol) in anh. THF (5 mL) under argon atmosphere at -78 °C, was added dropwise a solution of *n-*butyllithium (2.5 M in hexanes, 0.59 mL, 1.47 mmol) during 20 minutes. After the addition, the mixture was tempered to 0 °C using an ice bath. This solution was added carefully to the solution of the isocyanate from the previous step cooled to 0 °C, under argon atmosphere. The reaction mixture was stirred at room temperature overnight. Methanol (2 mL) was then added to quench any unreacted *n*-butyllithium. The solvents were evaporated under vacuum to give an orange gum (506 mg). This residue was dissolved in EtOAc (10 mL) and washed with 2N HCl solution (2 x 5 mL) and the organic layer was dried over anh. Na₂SO₄, filtered and concentrated under vacuum to obtain a white gum (241 mg). Column chromatography (SiO₂, DCM/Methanol mixtures) gave a white solid. Crystallization from hot DCM/Pentane provided pure 1-(1-(isopropylsulfonyl)piperidin-4-yl)-3-(9-methyl-5,6,8,9,10,11-hexahydro-7*H*-5,9:7,11-dimethanobenzo[9]annulen-7-yl)urea (66 mg, 13% yield) as a white solid.
mp 218-219 °C. IR (NaCl disk): 3364, 3061, 3012, 2945, 2919, 2853, 1709, 1638, 1553, 1493, 1453, 1360, 1319, 1305, 1265, 1248, 1232, 1133, 1091, 1045, 943, 880, 841, 759, 732, 665, 592, 555 cm⁻¹. Anal. Calcd for C₂₅H₃₇N₃O₃S: C 65.33, H 8.11, N 9.14. Found: C 65.41, H 8.31, N 8.93

### Example 44: 1-(1-benzylpiperidin-4-yl)-3-(9-methyl-5,6,8,9,10,11-hexahydro-7H-5,9:7,11-dimethanobenzo[9]annulen-7-yl)urea.

To a solution of 5-methyl-1,5,6,7-tetrahydro-1,5:3,7-dimethanobenzo[*e*]oxonin-3(2*H*)-amine hydrochloride (250 mg, 0.95 mmol) in DCM (4.5 mL) and saturated aqueous NaHCO₃ solution (3 mL) was added triphosgene (140 mg, 0.47 mmol). The biphasic mixture was stirred at room temperature for 30 minutes and then the two phases were separated and the organic layer was washed with brine (5 mL), dried over anh. Na₂SO₄, filtered and evaporated under vacuum to obtain 1-2 mL of a solution of the isocyanate in DCM. To this solution was added 1-(4-aminopiperidin-1-yl)ethan-1-one (216 mg, 1.13 mmol). The reaction mixture was stirred at room temperature for 24 h and the solvent was evaporated under vacuum to obtain a yellow gum. Column chromatography (SiO₂, DCM/Methanol mixtures) gave the title compound as a white solid (159 mg, 36% yield).
mp 106-107 °C. IR (NaCl disk): 3318, 3058, 3025, 2945, 2918, 2838, 2792, 2761, 1632, 1559, 1493, 1453, 1361, 1343, 1321, 1302, 1281, 1234, 1209, 1136, 1120, 1066, 1028, 909, 757, 733, 698 cm⁻¹. Anal. Calcd for C₂₉H₃₇N₃O·0.5 Methanol: C 77.09, H 8.55, N 9.14. Found: C 77.19, H 8.36, N 8.98.

### Reference example 45: N-(2-acetyl-9-methyl-5,6,8,9,10,11-hexahydro-7H-5,9:7,11-dimethanobenzo[9]annulen-7-yl)-2-chloroacetamide.

To a solution of 2-chloro-*N*-(9-methyl-5,6,8,9,10,11-hexahydro-7*H*-5,9:7,11-dimethanobenzo[9]annulen-7-yl)acetamide (2.0 g, 6.58 mmol) in DCM (50 mL) was added acetyl chloride (5.16 g, 65.8 mmol). Then, the mixture was treated with AlCl₃ (4.38 g, 32.9 mmol) and the resulting orange mixture was stirred for 1 h at room temperature. The solution was poured over ice (50 g) and saturated aqueous NaHCO₃ solution (40 mL) was added. After stirring 20 min, the mixture was extracted with DCM (3 x 50 mL) and the combined organic phases were dried over anh. Na₂SO₄ filtered and concentrated under vacuum to obtain a green gum (1.85 g). Column chromatography (SiO₂, Hexane/Ethyl Acetate mixtures) gave *N*-(2-acetyl-9-methyl-5,6,8,9,10,11-hexahydro-7*H*-5,9:7,11-dimethanobenzo[9]annulen-7-yl)-2-chloroacetamide (1.27 g, 56% yield) as a yellowish solid.

### Reference example 46: 1-(7-amino-9-methyl-6,7,8,9,10,11-hexahydro-5H-5,9:7,11-dimethanobenzo[9]annulen-2-yl)ethan-1-one hydrochloride.

A mixture of *N*-(2-acetyl-9-methyl-5,6,8,9,10,11-hexahydro-7*H*-5,9:7,11-dimethanobenzo[9]annulen-7-yl)-2-chloroacetamide (1.18 g, 3.43 mmol), thiourea (313 mg, 4.12 mmol), acetic acid (1.3 mL) and ethanol (6 mL) was stirred at reflux overnight. The mixture was tempered to room temperature and water (40 mL) and 10N NaOH solution (14 mL) were added. The mixture was extracted with EtOAc (3 x 50 mL) and the combined organic extracts were dried over anh. Na₂SO₄, filtered and concentrated under vacuum to obtain a yellow residue (980 mg) which was dissolved in EtOAc (5 mL) and an excess of HCl/Et₂O was added. The resulting suspension was filtrated obtaining a beige solid. This product was dissolved in DCM (50 mL) and washed with 5N NaOH solution (40 mL). The organic layer was dried over anh. Na₂SO₄, filtered and concentrated under vacuum to obtain a yellow residue which was dissolved in EtOAc (5 mL) and an excess of HCl/Et₂O was added. The resulting suspension was filtered obtaining 1-(7-amino-9-methyl-6,7,8,9,10,11-hexahydro-5*H*-5,9:7,11-dimethanobenzo[9]annulen-2-yl)ethan-1-one as its hydrochloride (758 mg, 73% yield) as a beige solid.

### Example 47: 1-(2-acetyl-9-methyl-5,6,8,9,10,11-hexahydro-7H-5,9:7,11-dimethano-benzo[9]annulen-7-yl)-3-(1-acetylpiperidin-4-yl)urea.

To a solution of 1-(7-amino-9-methyl-6,7,8,9,10,11-hexahydro-5*H*-5,9:7,11-dimethanobenzo[9]annulen-2-yl)ethan-1-one hydrochloride (300 mg, 0.98 mmol) in DCM (5 mL) and saturated aqueous NaHCO₃ solution (3.52 mL) was added triphosgene (145 mg, 0.49 mmol). The biphasic mixture was stirred at room temperature for 30 minutes and then the two phases were separated and the organic layer was washed with brine (5 mL), dried over anh. Na₂SO₄ filtered and evaporated under vacuum to obtain 1-2 mL of a solution of the isocyanate in DCM. To this solution was added 1-(4-aminopiperidin-1-yl)ethan-1-one (167 mg, 1.17 mmol). The reaction mixture was stirred at room temperature overnight and the solvent was evaporated under vacuum to obtain a yellow gum (483 mg). Column chromatography (SiO₂, DCM/Methanol mixtures) gave 1-(2-acetyl-9-methyl-5,6,8,9,10,11-hexahydro-7*H*-5,9:7,11-dimethanobenzo[9]annulen-7-yl)-3-(1-acetylpiperidin-4-yl)urea (324 mg, 76% yield).
mp 144-145 °C. IR (NaCl disk): 3363, 3005, 2918, 2861, 2239, 1679, 1619, 1552, 1453, 1426, 1361, 1320, 1272, 1229, 1203, 1137, 1106, 1057, 973, 950, 917, 830, 731, 645 cm⁻¹. Anal. Calcd for C₂₆H₃₅N₃O₃·0.15 C₅H₁₂ · 0.6 C₃H₆O: C 70.96, H 8.43, N 8.70. Found: C 70.83, H 8.60, N 8.88

### Example 48: 1-(1-acetylpiperidin-4-yl)-3-(9-methyl-2-nitro-5,6,8,9,10,11-hexahydro-7H-5,9:7,11-dimethanobenzo[9]annulen-7-yl)urea.

To a solution of 9-methyl-2-nitro-5,6,8,9,10,11-hexahydro-7*H*-5,9:7,11-dimethano-benzo[9]annulen-7-amine hydrochloride (600 mg, 1.94 mmol) in DCM (10 mL) saturated aqueous NaHCO₃ solution (10 mL) and triphosgene (213 mg, 0.718 mmol) were added. The biphasic mixture was stirred at room temperature for 30 minutes and then the two phases were separated and the organic layer was washed with brine (5 mL), dried over anh. Na₂SO₄ filtered and evaporated under vacuum to obtain 1-2 mL of a solution of the isocyanate in DCM. To this solution was added 1-(4-aminopiperidin-1-yl)ethan-1-one (331 mg, 2.33 mmol). The reaction mixture was stirred at room temperature overnight and the solvent was evaporated under vacuum to obtain a brown solid (840 mg). Column chromatography (SiO₂, DCM/Methanol mixtures) gave 1-(1-acetylpiperidin-4-yl)-3-(9-methyl-2-nitro-5,6,8,9,10,11-hexahydro-7*H*-5,9:7,11-dimethanobenzo[9]annulen-7-yl)urea (640 mg, 75% yield) as a yellowish solid.
mp 155-156 °C. IR (NaCl disk): 3360, 2918, 2237, 1619, 1552, 1522, 1454, 1345, 1322, 1266, 1230, 1164, 1137, 1081, 974, 949, 911, 865, 838, 798, 761, 731, 644 cm⁻¹. Anal. Calcd for C₂₄H₃₂N₄O₄: C 65.43, H 7.32, N 12.72. Found: C 65.22, H 7.45, N 12.56.

### Example 49: 1-(1-acetylpiperidin-4-yl)-3-(2-amino-9-methyl-5,6,8,9,10,11-hexa-hydro-7H-5,9:7,11-dimethanobenzo[9]annulen-7-yl)urea.

To a solution of 1-(1-acetylpiperidin-4-yl)-3-(9-methyl-2-nitro-5,6,8,9,10,11-hexahydro-7*H*-5,9:7,11-dimethanobenzo[9]annulen-7-yl)urea (260 mg, 0.59 mmol) in EtOH (17 ml) was added PtO₂ (20 mg). The mixture was hydrogenated at room temperature and atmospheric pressure for 8 days. The resulting suspension was filtered and the filtrate was evaporated under vacuum to obtain a dark brown solid (223 mg), which was dissolved in DCM (10 mL). To this solution, Et₂O was added and a white solid precipitated (140 mg) . Column chromatography (SiO₂, DCM/Methanol mixtures) gave a white solid (82 mg, 34% yield).
mp 150-151 °C. IR (NaCl disk): 3344, 3006, 2905, 2853, 1614, 1556, 1505, 1454, 1360, 1344, 1320, 1303, 1266, 1229, 1194, 1162, 1136, 1060, 974, 868, 820, 734 cm⁻¹. HRMS-ESI+ m/z [M+H]⁺ calcd for [C₂₄H₃₄N₄O₂+H]⁺: 411.2755, found: 411.2756.

### Example 50: tert-butyl 4-(2-((9-methyl-5,6,8,9,10,11-hexahydro-7H-5,9:7,11-dimethanobenzo[9]annulen-7-yl)amino)-2-oxoethyl)piperidine-1-carboxylate.

To a suspension of 9-methyl-5,6,8,9,10,11-hexahydro-7*H*-5,9:7,11-dimethanobenzo[9]annulen-7-amine hydrochloride (500 mg, 1.89 mmol) in EtOAc (5 mL), 2-(1-(*tert*-butoxycarbonyl)piperidin-4-yl)acetic acid (461 mg, 1.89 mmol), HOBt (384 mg, 2.84 mmol), EDC·HCl (440 mg, 2.84 mmol) and Et₃N (767 mg, 7.58 mmol) were added. The mixture was stirred at room temperature for 24 h. Water (10 mL) and DCM (20 mL) were added to the resulting suspension and the 2 phases were separated. The organic phase was washed with saturated aqueous NaHCO₃ solution (1x10 mL), brine (1x10 ml), 2N HCl solution (1x10 mL) and 2N NaOH (1x10 mL), dried over anh. Na₂SO₄, filtered and concentrated under vacuum to give a yellow solid (515 mg, 60% yield).
¹H-NMR (400 MHz, CDCl₃) δ: 0.92 (s, 3H), 1.11 (dq, *J* = 4.4 Hz, *J'* = 11.6 Hz, 2H), 1.4 (s, 9H), 1.54 (d, *J* = 13.6 Hz, 2H), 1.63-1.68 (complex signal, 4H), 1.84 (s, 2H), 1.91 (m, 1H), 1.97 (s, 2H), 2.0 (d, *J* = 12.8 Hz, 2H), 2.14-2.18 (complex signal, 2H), 2.69 (t, *J* = 13.2 Hz, 2H), 3.06 (t, *J* = 6Hz, 2H), 4.06 (broad signal, 2H), 5.14 (s, 1H), 7.02-7.08 (complex signal, 4H).

### Example 51: N-(9-methyl-5,6,8,9,10,11-hexahydro-7H-5,9:7,11-dimethanobenzo [9]annulen-7-yl)-2-(piperidin-4-yl)acetamide.

To a solution of *tert*-butyl 4-(2-((9-methyl-5,6,8,9,10,11-hexahydro-7*H*-5,9:7,11-dimethanobenzo[9]annulen-7-yl)amino)-2-oxoethyl)piperidine-1-carboxylate (250 mg, 0.55 mmol) in DCM (4 mL) was added 4M HCI in 1,4-dioxane (0.5 ml). The reaction mixture was stirred at room temperature for 3 days. Then, the solvent was evaporated under vacuum and the residue was dissolved in DCM (10 mL) and washed with 5N NaOH solution, dried over anh. Na₂SO₄, filtered and concentrated under vacuum to give a yellow solid (189 mg, 97% yield).
¹H-NMR (400 MHz, CDCl₃) δ: 0.91 (s, 3H), 1.12 (dq, *J* = 4 Hz, *J'* = 12.0 Hz, 2H), 1.53 (d, *J* = 13.2 Hz, 2H), 1.62-1.71 (complex signal, 4H), 1.84 (s, 2H), 1.88 (m, 1H), 1.95-2.01 (complex signal, 4H), 2.14-2.19 (complex signal, 2H), 2.6 (dt, *J* = 2.8 Hz, *J'* = 12.0 Hz, 2H), 3.00-3.07 (complex signal, 4H), 5.15 (s, 1H), 7.02-7.09 (complex signal, 4H).

### Example 52: 2-[1-(isopropylsulfonyl)piperidin-4-yl]-N-(9-methyl-5,6,8,9,10,11-hexahydro-7H-5,9:7,11-dimethanobenzo[9]annulen-7-yl)acetamide.

To a solution of *N*-(9-methyl-5,6,8,9,10,11-hexahydro-7*H*-5,9:7,11-dimethanobenzo[9]annulen-7-yl)-2-(piperidin-4-yl)acetamide (185 mg, 0.52 mmol) in DCM (5 mL) was added Et₃N (63 mg, 0.63 mmol). The mixture was cooled down to 0 °C and propane-2-sulfonyl chloride (74 mg, 0.52 mmol) was added dropwise. Then, the reaction mixture was stirred at room temperature overnight and quenched by addition of 2N HCI solution (3 mL). The two phases were separated and the aqueous phase was extracted with EtOAc (2 x 20 mL). The combined organic phases were washed with 5N NaOH solution, dried over anh. Na₂SO₄, filtered and concentrated under vacuum to give a yellow solid. Column chromatography (SiO₂, Hexane/Ethyl Acetate mixtures) gave a white solid (145 mg, 60% yield). The analytical sample was obtained by crystallization from hot EtOAc (76 mg).
mp 172 -173 °C. IR (NaCl disk): 3365, 3319, 3058, 3017, 2916, 2852, 1648, 1536, 1493, 1451, 1361, 1322, 1308, 1264, 1167, 1137, 1044, 1011, 993, 944, 904, 880, 800, 758, 731, 701, 665 cm⁻¹. Anal. Calcd for C₂₆H₃₈N₂O₃S·0.25 Methanol: C 67.56, H 8.42, N 6.00. Found: C 67.75 H 8.62, N 5.74.

### Example 53: 2-(1-acetylpiperidin-4-yl)-N-(9-methyl-5,6,8,9,10,11-hexahydro-7H-5,9:7,11-dimethanobenzo[9]annulen-7-yl)acetamide.

To a solution of *N*-(9-methyl-5,6,8,9,10,11-hexahydro-7*H*-5,9:7,11-dimethanobenzo[9]annulen-7-yl)-2-(piperidin-4-yl)acetamide (200 mg, 0.57 mmol) in anh. DCM (5 mL) under argon atmosphere was added anh. Et₃N (69 mg, 0.68 mmol). The mixture was cooled down to 0°C and acetyl chloride (45 mg, 0.57 mmol) was added dropwise. Then, the reaction mixture was stirred at room temperature overnight and quenched by addition of 2N HCI solution (3 mL). The two phases were separated and the aqueous layer was extracted with EtOAc (2 x 20 mL). The combined organic phases were washed with 2N NaOH solution, dried over anh. Na₂SO₄, filtered and concentrated under vacuum. Column chromatography (SiO₂, Hexane/Ethyl Acetate mixtures) gave a white solid (134 mg, 48% yield).
mp 85-86 °C. IR (NaCl disk): 3314, 3060, 3016, 2915, 2859, 2239, 1630, 1544, 1492, 1450, 1361, 1303, 1273, 1196, 1164, 1137, 1096, 1048 cm⁻¹. Anal. Calcd for C₂₅H₃₄N₂O₂·0.15 DCM: C 74.17, H 8.49, N 6.88. Found: C 74.31, H 8.73, N 6.72.

### Example 54: 1-(9-methyl-6,7,8,9,10,11-hexahydro-5H-5,9:7,11-dimethanobenzo[9] annulen-7-yl)-3-(2,3,4-trifluorophenyl)urea.

To a solution of 5-methyl-1,5,6,7-tetrahydro-1,5:3,7-dimethanobenzo[*e*]oxonin-3(2*H*)-amine (273 mg, 1.2 mmol) in anhydrous DCM (10 mL), 2,3,4-trifluorophenylisocyanate (147 mg, 1.0 mmol) and triethylamine (0.55 mg, 4 mmol) were added. The reaction mixture was stirred at room temperature overnight. Then the solvent was removed under vacuum. Column chromatography (SiO₂, Hexane/Ethyl Acetate mixture) of the crude and concentration under vacuum of the appropriate fractions gave the urea (38 mg, 13% yield) as a white solid.
mp 206-207 °C. IR (ATR): 3331, 2903, 2839, 1654, 1556, 1510, 1473, 1361, 1344, 1290, 1237, 1174, 1101, 1038, 1019, 1004, 800, 756, 690, 669, 625 cm⁻¹. Anal. Calcd for C₂₃H₂₃F₃N₂O: C 68.99, H 5.79, N 7.00. Found: C 68.94, H 5.92, N 6.71.

### Example 55: 1-(5-methy)-1,5,6,7-tetrahydro-1,5:3,7-dimethanobenzo[e]oxonin-3(2H)-yl)-3-(2,3,4-trifluorophenyl)urea.

To a solution of 5-methyl-1,5,6,7-tetrahydro-1,5:3,7-dimethanobenzo[*e*]oxonin-3(2*H*)-amine (275 mg, 1.2 mmol) in anhydrous DCM (10 mL), 2,3,4-trifluorophenylisocyanate (147 mg, 1.0 mmol) and and triethylamine (0.55 mg, 4 mmol) were added. The reaction mixture was stirred at room temperature overnight. Then the solvent was removed under vacuum. The desired urea was obtained as a white solid (205 mg, 54% yield).
mp 257-259 °C. IR (ATR): 3295, 3241, 3118, 2916, 2173, 1693, 1620, 1564, 1510, 1493, 1468, 1462, 1356, 1345, 1320, 1302, 1286, 1273, 1254, 1229, 1210, 1181, 1167, 1111, 1091, 1074, 1049, 1035, 1008, 999, 958, 906, 820, 812, 763, 646 cm⁻¹. Anal. Calcd for C₂₂H₂₁F₃N₂O₂·0-1H₂O: C 65.37, H 5.29, N 6.93. Found: C 65.18, H 5.31,N 6.73. HRMS-ESI+ m/z [M+H]⁺ calcd for [C₂₂H₂₁F₃N₂O₂+H]⁺: 403.1633, found: 403.1631.

### Example 56: 2-(1-benzylpiperidin-4-yl)-N-(9-methyl-5,6,8,9,10,11-hexahydro-7H-5,9:7,11-dimethanobenzo[9]annulen-7-yl)acetamide.

To a suspension of 9-methyl-5,6,8,9,10,11-hexahydro-7*H*-5,9:7,11-dimethanobenzo[9]annulen-7-amine hydrochloride (250 mg, 0.95 mmol) in EtOAc (5 mL), 2-(1-benzylpiperidin-4-yl)acetic acid hydrochloride (255 mg, 0.95 mmol), HOBt (192 mg, 1.42 mmol), EDC·HCl (220 mg, 1.42 mmol) and Et₃N (480 mg, 4.74 mmol) were added. The mixture was stirred at room temperature for 24 h. Water (10 mL) and DCM (10 mL) were added to the resulting suspension and the 2 phases were separated. The organic phase was washed with saturated aqueous NaHCO₃ solution (1x10 mL), brine (1x10 ml), dried over anh. Na₂SO₄, filtered and concentrated under vacuum to give a yellow gum (479 mg). Column chromatography (SiO₂, DCM/Methanol mixtures) gave a white solid (280 mg, 67% yield). The analytical sample was obtained by crystallization from hot EtOAc and Et₂O (124 mg).
mp 145-146 °C. IR (NaCl disk): 3302, 3060, 3024, 2917, 2841, 2798, 2755, 1641, 1544, 1493, 1452, 1361, 1342, 1309, 1279, 1211, 1184, 1143, 1077, 1008, 974, 943, 916, 794, 756, 737, 697 cm⁻¹. HRMS-ESI+ m/z [M+H]⁺ calcd for [C₃₀H₃₈N₂O+H]⁺: 443.3057, found: 443.3061.

### Reference example 57: tert-butyl (1-propionylpiperidin-4-yl)carbamate.

To a solution of *tert*-butyl piperidin-4-ylcarbamate (500 mg, 2.49 mmol) in anh. THF (5 mL) was added Et₃N (252 mg, 2.49 mmol). The mixture was cooled down to 0 °C with an ice bath and then propionyl chloride (230 mg, 2.49 mmol) was added dropwise. The reaction mixture was stirred at room temperature for 2h. The suspension was filtrated and the filtered was evaporated to obtain the carbamate as a yellowish solid (661 mg, quantitative yield).

### Reference example 58: 1-(4-aminopiperidin-1-yl)propan-1-one.

To a solution of *tert*-butyl (1-propionylpiperidin-4-yl)carbamate (660 g, 2.57 mmol) in DCM (3 mL) 4 M HCI in 1,4-dioxane (2 mL) was added. The mixture was stirred at room temperature overnight and the solvents were evaporated under vacuum. The residue was then dissolved in DCM (5 mL) and washed with 5N NaOH solution (5 mL). The organic layer was dried over anh. Na₂SO₄, filtered and concentrated under vacuum to give 1-(4-aminopiperidin-1-yl)propan-1-one (335 mg, 83% yield) as a yellow oil.

### Example 59: 1-(9-methyl-5,6,8,9,10,11-hexahydro-7H-5,9:7,11-dimethanobenzo[9] annulen-7-yl)-3-(1-propionylpiperidin-4-yl)urea.

To a solution of 9-methyl-5,6,8,9,10,11-hexahydro-7*H*-5,9:7,11-dimethanobenzo[9] annulen-7-amine hydrochloride (464 mg, 1.76 mmol) in DCM (10 mL) saturated aqueous NaHCO₃ solution (10 mL) and triphosgene (193 mg, 0.65 mmol) were added. The biphasic mixture was stirred at room temperature for 30 minutes and then the two phases were separated and the organic layer was washed with brine (5 mL), dried over anh. Na₂SO₄, filtered and evaporated under vacuum to obtain 1-2 mL of a solution of the isocyanate in DCM. To this solution was added 1-(4-aminopiperidin-1-yl)propan-1-one (350 mg, 2.11 mmol). The reaction mixture was stirred at room temperature overnight and the solvent was evaporated under vacuum to obtain a white solid (741 mg). Column chromatography (SiO₂, DCM/Methanol mixtures) gave 1-(9-methyl-5,6,8,9,10,11-hexahydro-7H-5,9:7,11-dimethanobenzo[9]annulen-7-yl)-3-(1-propionylpiperidin-4-yl)urea (597 mg, 83% yield) as a white solid. The analytical sample was obtained by crystallization from hot EtOAc and DCM (300 mg).
mp 207-208 °C. IR (NaCl disk): 3357, 2917, 2858, 1644, 1620, 1555, 1493, 1449, 1360, 1344, 1318, 1263, 1221, 1131, 1067, 1023, 971, 948, 758 cm⁻¹. Anal. Calcd for C₂₅H₃₅N₃O₂·0.15 EtOAc: C 72.73, H 8.63, N 9.94. Found: C 72.65, H 8.49, N 9.82.

### Example 60: 1-(1-(4-acetylphenyl)piperidin-4-yl)-3-(9-methyl-5,6,8,9,10,11-hexahydro-7H-5,9:7,11-dimethanobenzo[9]annulen-7-yl)urea.

To a solution of 9-methyl-5,6,8,9,10,11-hexahydro-7*H*-5,9:7,11-dimethanobenzo[9] annulen-7-amine hydrochloride (241 mg, 0.95 mmol) in DCM (5 mL) saturated aqueous NaHCO₃ solution (5 mL) and triphosgene (104 mg, 0.35 mmol) were added. The biphasic mixture was stirred at room temperature for 30 minutes and then the two phases were separated and the organic layer was washed with brine (5 mL), dried over anh. Na₂SO₄, filtered and evaporated under vacuum to obtain 1-2 mL of a solution of the isocyanate in DCM. To this solution was added 1-(4-(4-aminopiperidin-1-yl)phenyl)ethan-1-one (250 mg, 1.15 mmol, prepared following the procedure reported in WO2007016496). The reaction mixture was stirred at room temperature overnight and the solvent was evaporated under vacuum to obtain an orange solid (475 mg). Column chromatography (SiO₂, Hexane/Ethyl Acetate mixtures) gave 1-(1-(4-acetylphenyl)piperidin-4-yl)-3-(9-methyl-5,6,8,9,10,11-hexahydro-7*H*-5,9:7,11-dimethanobenzo[9]annulen-7-yl)urea (120 mg, 27% yield) as a yellowish solid.
mp 211-212 °C. IR (NaCl disk): 3357, 2919, 2844, 1666, 1633, 1596, 1552, 1518, 1493, 1452, 1427, 1389, 1359, 1306, 1281, 1224, 1193, 1128, 1068, 956, 915, 825, 758 cm⁻¹. HRMS-ESI+ m/z [M+H]⁺ calcd for [C₃₀H₃₇N₃O₂+H]⁺: 472.2959, found: 472.2962.

### Example 61: 1-(9-methy)-5,6,8,9,10,11-hexahydro-7H-5,9:7,11-dimethanobenzo[9] annulen-7-yl)-3-(1-(tetrahydro-2H-pyran-4-carbonyl)piperidin-4-yl)urea.

To a solution of 9-methyl-5,6,8,9,10,11-hexahydro-7*H*-5,9:7,11-dimethanobenzo[9] annulen-7-amine hydrochloride (258 mg, 0.98 mmol) in DCM (4 mL) saturated aqueous NaHCO₃ solution (4 mL) and triphosgene (107 mg, 0.36 mmol) were added. The biphasic mixture was stirred at room temperature for 30 minutes and then the two phases were separated and the organic layer was washed with brine (2 mL), dried over anh. Na₂SO₄, filtered and evaporated under vacuum to obtain 1-2 mL of a solution of the isocyanate in DCM. To this solution was added (4-aminopiperidin-1-yl)(tetrahydro-2*H*-pyran-4-yl)methanone (215 mg, 1.10 mmol). The reaction mixture was stirred at room temperature overnight and the solvent was evaporated under vacuum to obtain a yellow residue (534 mg). Column chromatography (SiO₂, DCM/Methanol mixtures) gave 1-(9-methyl-5,6,8,9,10,11-hexahydro-7*H*-5,9:7,11-dimethanobenzo[9]annulen-7-yl)-3-(1-(tetrahydro-2*H*-pyran-4-carbonyl)piperidin-4-yl)urea (207 mg, 45% yield) as a white solid.
mp 224-225 °C. IR (NaCl disk): 3356, 3064, 2945, 2919, 2850, 1639, 1613, 1552, 1493, 1446, 1360, 1344, 1320, 1278, 1261, 1238, 1211, 1126, 1089, 1068, 1018, 983, 941, 874, 818, 758, 733 cm⁻¹. HRMS-ESI+ m/z [M+H]⁺ calcd for [C₂₈H₃₉N₃O₃+H]⁺: 466.3064, found: 466.3065.

### Example 62: 1-(1-(2-fluorobenzoyl)piperidin-4-yl)-3-(9-methyl-5,6,8,9,10,11-hexahydro-7H-5,9:7,11-dimethanobenzo[9]annulen-7-yl)urea.

To a solution of 9-methyl-5,6,8,9,10,11-hexahydro-7*H*-5,9:7,11-dimethanobenzo[9]annulen-7-amine hydrochloride (247 mg, 0.93 mmol) in DCM (4 mL) saturated aqueous NaHCO₃ solution (4 mL) and triphosgene (103 mg, 0.36 mmol) were added. The biphasic mixture was stirred at room temperature for 30 minutes and then the two phases were separated and the organic layer was washed with brine (3 mL), dried over anh. Na₂SO₄, filtered and evaporated under vacuum to obtain 1-2 mL of a solution of the isocyanate in DCM. To this solution was added (4-aminopiperidin-1-yl)(2-fluorophenyl)methanone (250 mg, 1.12 mmol). The reaction mixture was stirred at room temperature overnight and the solvent was evaporated under vacuum to obtain a white solid (486 mg). Column chromatography (SiO₂, DCM/Methanol mixtures) gave 1-(1-(2-fluorobenzoyl)piperidin-4-yl)-3-(9-methyl-5,6,8,9,10,11-hexahydro-7*H*-5,9:7,11-dimethanobenzo[9]annulen-7-yl)urea (285 mg, 45% yield) as a white solid.
mp 265-266 °C. IR (NaCl disk): 3368, 2920, 2854, 1614, 1549, 1492, 1452, 1364, 1318, 1282, 1222, 1122, 1089, 1029, 974, 948, 817, 755 cm⁻¹. HRMS-ESI+ m/z [M+H]⁺ calcd for [C₂₉H₃₄FN₃O₂+H]⁺: 476.2708, found: 476.2711.

### Example 63: 1-((1R,3s,5S)-8-benzyl-8-azabicyclo[3.2.1]octan-3-yl)-3-(9-methyl-5,6, 8,9,10,11-hexahydro-7H-5,9:7,11-dimethanobenzo[9]annulen-7-yl)urea.

To a solution of 9-methyl-5,6,8,9,10,11-hexahydro-7*H*-5,9:7,11-dimethanobenzo[9] annulen-7-amine hydrochloride (253 mg, 0.96 mmol) in DCM (4 mL) saturated aqueous NaHCO₃ solution (4 mL) and triphosgene (105 mg, 0.35 mmol) were added. The biphasic mixture was stirred at room temperature for 30 minutes and then the two phases were separated and the organic layer was washed with brine (3 mL), dried over anh. Na₂SO₄, filtered and evaporated under vacuum to obtain 1-2 mL of a solution of the isocyanate in DCM. To this solution was added (1R,3s,5S)-8-benzyl-8-azabicyclo[3.2.1]octan-3-amine (250 mg, 1.15 mmol). The reaction mixture was stirred at room temperature overnight and the solvent was evaporated under vacuum to obtain a yellow gum (498 mg). Column chromatography (SiO₂, DCM/Methanol mixtures) gave 1-((1R,3s,5S)-8-benzyl-8-azabicyclo[3.2.1]octan-3-yl)-3-(9-methyl-5,6,8,9,10,11-hexahydro-7*H*-5,9:7,11-dimethanobenzo[9]annulen-7-yl)urea (293 mg, 65% yield) as a white solid. The analytical sample was obtained by crystallization from hot mixture EtOAc:Et₂O (187 mg).
mp 100-101 °C. IR (NaCl disk): 3319, 3022, 2944, 2919, 2843, 1632, 1557, 1493, 1452, 1344, 1321, 1304, 1279, 1263, 1235, 1164, 1122, 1056, 1027, 756, 729, 696 cm⁻¹. HRMS-ESI+ m/z [M+H]⁺ calcd for [C₃₁H₃₉N₃O+H]⁺: 470.3166, found: 470.3168.

### Example 64: 1-(1-acetylpiperidin-4-yl)-3-(2-fluoro-9-methyl-5,6,8,9,10,11-hexahydro-7H-5,9:7,11-dimethanobenzo[9]annulen-7-yl)urea.

To a solution of 2-fluoro-9-methyl-5,6,8,9,10,11-hexahydro-7*H*-5,9:7,11-dimethanobenzo[9]annulen-7-amine hydrochloride (150 mg, 0.53 mmol) in DCM (3 mL) saturated aqueous NaHCO₃ solution (3 mL) and triphosgene (59 mg, 0.20 mmol) were added. The biphasic mixture was stirred at room temperature for 30 minutes and then the two phases were separated and the organic layer was washed with brine (3 mL), dried over anh. Na₂SO₄, filtered and evaporated under vacuum to obtain 1-2 mL of a solution of the isocyanate in DCM. To this solution was added 1-(4-aminopiperidin-1-yl)ethan-1-one (91 mg, 0.64 mmol). The reaction mixture was stirred at room temperature overnight and the solvent was evaporated under vacuum to obtain a yellowish oil (165 mg). Column chromatography (SiO₂, DCM/Methanol mixtures) gave 1-(1-acetylpiperidin-4-yl)-3-(2-fluoro-9-methyl-5,6,8,9,10,11-hexahydro-7*H*-5,9:7,11-dimethanobenzo[9]annulen-7-yl)urea (103 mg, 49% yield) as a white solid.
mp 269-270 °C. IR (NaCl disk): 3357, 2919, 2856, 1644, 1620, 1555, 1499, 1453, 1361, 1342, 1320, 1228, 1153, 1138, 1064, 967, 863, 818 cm⁻¹. HRMS-ESI+ m/z [M+H]⁺ calcd for [C₂₄H₃₂FN₃O₂+H]⁺: 414.2551, found: 414.2553.

### Example 65: 1-(1-acetylpiperidin-4-yl)-3-(2-methoxy-9-methyl-5,6,8,9,10,11-hexahydro-7H-5,9:7,11-dimethanobenzo[9]annulen-7-yl)urea.

To a solution of 2-methoxy-9-methyl-5,6,8,9,10,11-hexahydro-7*H*-5,9:7,11-dimethanobenzo[9]annulen-7-aminehydrochloride (150 mg, 0.51 mmol) in DCM (3 mL) saturated aqueous NaHCO₃ solution (3 mL) and triphosgene (56 mg, 0.19 mmol) were added. The biphasic mixture was stirred at room temperature for 30 minutes and then the two phases were separated and the organic layer was washed with brine (3 mL), dried over anh. Na₂SO₄, filtered and evaporated under vacuum to obtain 1-2 mL of a solution of the isocyanate in DCM. To this solution was added 1-(4-aminopiperidin-1-yl)ethan-1-one (87 mg, 0.61 mmol). The reaction mixture was stirred at room temperature overnight and the solvent was evaporated under vacuum to obtain a brown oil (256 mg). Column chromatography (SiO₂, DCM/Methanol mixtures) gave 1-(1-acetylpiperidin-4-yl)-3-(2-methoxy-9-methyl-5,6,8,9,10,11-hexahydro-7*H*-5,9:7,11-dimethanobenzo[9]annulen-7-yl)urea (121 mg, 56 % yield) as a white solid.
mp 116-117 °C. IR (NaCl disk): 3359, 2905, 2861, 1644, 1619, 1551, 1501, 1452, 1360, 1343, 1319, 1267, 1227, 1153, 1136, 1042, 973, 807, 736 cm⁻¹. HRMS-ESI+ m/z [M+H]⁺ calcd for [C₂₅H₃₅N₃O₃+H]⁺: 426.2571, found: 4426.2760.

### Example 66: 1-(1-acetylpiperidin-4-yl)-3-(1-fluoro-9-methyl-5,6,8,9,10,11-hexahydro-7H-5,9:7,11-dimethanobenzo[9]annulen-7-yl)urea.

To a solution of 1-fluoro-9-methyl-5,6,8,9,10,11-hexahydro-7*H*-5,9:7,11-dimethanobenzo[9]annulen-7-amine hydrochloride (150 mg, 0.53 mmol) in DCM (3 mL) saturated aqueous NaHCO₃ solution (3 mL) and triphosgene (58 mg, 0.20 mmol) were added. The biphasic mixture was stirred at room temperature for 30 minutes and then the two phases were separated and the organic layer was washed with brine (3 mL), dried over anh. Na₂SO₄, filtered and evaporated under vacuum to obtain 1-2 mL of a solution of the isocyanate in DCM. To this solution was added 1-(4-aminopiperidin-1-yl)ethan-1-one (91 mg, 0.64 mmol). The reaction mixture was stirred at room temperature overnight and the solvent was evaporated under vacuum to obtain a yellow oil (320 mg). Column chromatography (SiO₂, DCM/Methanol mixtures) gave 1-(1-acetylpiperidin-4-yl)-3-(1-fluoro-9-methyl-5,6,8,9,10,11-hexahydro-7*H*-5,9:7,11-dimethanobenzo[9]annulen-7-yl)urea (160 mg, 73% yield) as a white solid.
mp 122-123 °C. IR (NaCl disk): 3351, 2944, 2918, 2861, 1642, 1618, 1555, 1462, 1362, 1321, 1238, 1137, 1066, 976, 885, 798, 749 cm⁻¹. HRMS-ESI+ m/z [M+H]⁺ calcd for [C₂₄H₃₂FN₃O₂+H]⁺: 414.2551, found: 414.2554.

### Example 67: 1-(1-acetylpiperidin-4-yl)-3-(2,3-dimethoxy-9-methyl-5,6,8,9,10,11-hexahydro-7H-5,9:7,11-dimethanobenzo[9]annulen-7-yl)urea.

To a solution of 2,3-dimethoxy-9-methyl-5,6,8,9,10,11-hexahydro-7*H*-5,9:7,11-dimethanobenzo[9]annulen-7-amine hydrochloride (150 mg, 0.46 mmol) in DCM (3 mL) saturated aqueous NaHCO₃ solution (3 mL) and triphosgene (51 mg, 0.17 mmol) were added. The biphasic mixture was stirred at room temperature for 30 minutes and then the two phases were separated and the organic layer was washed with brine (3 mL), dried over anh. Na₂SO₄, filtered and evaporated under vacuum to obtain 1-2 mL of a solution of the isocyanate in DCM. To this solution was added 1-(4-aminopiperidin-1-yl)ethan-1-one (79 mg, 0.55 mmol). The reaction mixture was stirred at room temperature overnight and the solvent was evaporated under vacuum to obtain a yellow oil (334 mg). Column chromatography (SiO₂, DCM/Methanol mixtures) gave 1-(1-acetylpiperidin-4-yl)-3-(2,3-dimethoxy-9-methyl-5,6,8,9,10,11-hexahydro-7*H-*5,9:7,11-dimethanobenzo[9]annulen-7-yl)urea (168 mg, 80 % yield) as a white solid. mp 127-128 °C. IR (NaCl disk): 3365, 3052, 2913, 2862, 2834, 1643, 1616, 1553, 1516, 1452, 1360, 1343, 1320, 1293, 1252, 1232, 1168, 1137, 1092, 1019, 974, 863, 801, 734 cm⁻¹. HRMS-ESI+ m/z [M+H]⁺ calcd for [C₂₆H₃₇N₃O₄+H]⁺: 456.2857, found: 456.2859.

### Example 68: 1-(1-acetylpiperidin-4-yl)-3-(5,8,9,10-tetrahydro-5,8:7,10-dimethanobenzo[8]annulen-7(6H)-yl)urea.

To a solution of 5,8,9,10-tetrahydro-5,8:7,10-dimethanobenzo[8]annulen-7(6*H*)-amine hydrochloride (57 mg, 0.24 mmol) in DCM (1 mL) saturated aqueous NaHCO₃ solution (1 mL) and triphosgene (27 mg, 0.09 mmol) were added. The biphasic mixture was stirred at room temperature for 30 minutes and then the two phases were separated and the organic layer was washed with brine (1 mL), dried over anh. Na₂SO₄, filtered and evaporated under vacuum to obtain 1-2 mL of a solution of the isocyanate in DCM. To this solution was added 1-(4-aminopiperidin-1-yl)ethan-1-one (41 mg, 0.29 mmol). The reaction mixture was stirred at room temperature overnight and the solvent was evaporated under vacuum to obtain a brown gum (93 mg). Column chromatography (SiO₂, DCM/Methanol mixtures) gave 1-(1-acetylpiperidin-4-yl)-3-(5,8,9,10-tetrahydro-5,8:7,10-dimethanobenzo[8]annulen-7(6*H*)-yl)urea (47 mg, 53 % yield) as a white solid. mp 98-99 °C. IR (NaCl disk): 3359, 3013, 2927, 2856, 2239, 1621, 1556, 1449, 1372, 1334, 1318, 1268, 1238, 1225, 1192, 1153, 1107, 1081, 1048, 1041, 972, 920, 756, 730 cm⁻¹. HRMS-ESI+ m/z [M+H]⁺ calcd for [C₂₂H₂₉N₃O₂+H]⁺: 4368.2333, found: 368.2331.

### Example 69: 1-(benzo[d]thiazol-2-yl)-3-(9-methoxy-5,6,8,9,10,11-hexahydro-7H-5,9:7,11-dimethanobenzo[9]annulen-7-yl)urea.

To a solution of 9-methoxy-5,6,8,9,10,11-hexahydro-7*H*-5,9:7,11-dimethanobenzo[9]annulen-7-amine (250 mg, 1.03 mmol) in DCM (3 mL) saturated aqueous NaHCO₃ solution (3 mL) and triphosgene (113 mg, 0.38 mmol) were added. The biphasic mixture was stirred at room temperature for 30 minutes and then the two phases were separated and the organic layer was washed with brine (3 mL), dried over anh. Na₂SO₄, filtered and evaporated under vacuum to obtain 1-2 mL of a solution of the isocyanate in DCM.

To a solution of benzo[d]thiazol-2-amine (141 mg, 0.94 mmol) in anh. THF (8 mL) under argon atmosphere at -78 °C, was added dropwise a solution of *n*-butyllithium (2.5 M in hexanes, 0.38 mL, 0.94 mmol) during 20 minutes. After the addition, the mixture was tempered to 0 °C using an ice bath. This solution was added carefully to the solution of the isocyanate from the previous step cooled to 0 °C, under argon atmosphere. The reaction mixture was stirred at room temperature overnight. Methanol (3 mL) was then added to quench any unreacted *n*-butyllithium. The solvents were evaporated under vacuum to give a yellow solid (531 mg). Column chromatography (SiO₂, Hexane/Ethyl Acetate mixtures) gave a 1-(benzo[*d*]thiazol-2-yl)-3-(9-methoxy-5,6,8,9,10,11-hexahydro-7*H*-5,9:7,11-dimethanobenzo[9]annulen-7-yl)urea (65 mg, 15% yield) as a white solid.
mp 247-248 °C. IR (NaCl disk): 2926, 2851, 1712, 1675, 1593, 1537, 1445, 1358, 1268, 1217, 1556, 1116, 1080, 1044, 1015, 910, 845 cm^{-1.} HRMS-ESI+ m/z [M+H]⁺ calcd for [C₂₄H₂₅N₃O₂S+H]⁺: 420.1740, found: 368.2331.

### Example 70: In vitro determination of sEH inhibition activity

The following fluorescent assay was used for determination of the sEH inhibition activity (IC₅₀), with the substrate and comparative control compound (TPPU) indicated below.

**Substrate:** cyano(6-methoxynaphthalen-2-yl)methyl 2-(3-phenyloxiran-2-yl)acetate (PHOME; from Cayman Chemical, item number 10009134; CAS 1028430-42-3); cf. N.M. Wolf et al., Anal. Biochem. 2006, vol. 355, pp. 71-80.

**TPPU:** *N*-[1-(1-Oxopropyl)-4-piperidinyl]-*N*'-[4-(trifluoromethoxy)phenyl]urea.

### Solutions:

- Assay buffer: Bis/Tris HCI 25 mM pH 7.0 containing 0.1 mg/mL of bovine serum albumin (BSA).
- PHOME at 200 µM in DMSO.
- Solution of recombinant human sEH (hsEH) (Cayman Chemical, item number 10011669), diluted with assay buffer.
- Inhibitors dissolved in DMSO at appropriated concentrations.

**Protocol:** In a black 96-well plate (Greiner Bio-One, item number 655900), fill the background wells with 90 µL and the positive control and inhibitor wells with 85 µL of assay buffer. Add 5 µL of DMSO to background and positive control wells, and then add 5 µL of inhibitor solution in inhibitor wells. Add 5 µL of the solution of hsEH to the positive control and inhibitor wells and stir the mixture. Prepare a 1/21 dilution of the solution of PHOME with assay buffer according to final volume required, and then add 105 µL of each well. Shake carefully the plate for 10 seconds and incubate for 5 minutes at room temperature. Read the appearance of fluorescence with excitation wavelength: 337 nm, and emission wavelength: 460 nm (FLUOStar OPTIMA microplate reader, BMG). The intensity of fluorescence was used to analyze and calculate the IC₅₀ values. Results were obtained by regression analysis from at least three data points in a linear region of the curve. IC₅₀ values are average of minimum three independent replicates.

**Table 1: human sEH inhibition activity (IC₅₀, nM) of selected compounds (I)^{a}**

| Ex. | TPPU | 34 | 36 | 37 | 39 | 40 | 43 | 44 | 47 | 48 | 49 | 52 | 54 | 55 | 59 | 60 |
|---|---|---|---|---|---|---|---|---|---|---|---|---|---|---|---|---|
| IC₅₀ | A | A | C | A | B | A | A | A | A | A | B | B | A | B | A | A |

| | | | | | | | | | | | | | | | | |
|---|---|---|---|---|---|---|---|---|---|---|---|---|---|---|---|---|
| ^{a} A means that IC₅₀ is lower than 10 nM, B means that IC₅₀ is at least 10 nM but less than 50 nM and C means that IC₅₀ is at least 50 nM but less than 100 nM. | | | | | | | | | | | | | | | | |

### LIST OF REFERENCES

### Non-patent literature cited in the description

1.Abstracts of Papers, 241st ACS National Meeting & Exposition, Anaheim, CA, United States, March 27-31, 2011 (2011), MEDI-92
2.ACS Chem Biol. 2018 Jan 19; 13:45-52
3.Am J Physiol Renal Physiol. 2013 Jan 15;304(2):F168-76
4.Am J Physiol Renal Physiol. 2014 Oct 15;307(8):F971-80
5.Am J Respir Cell Mol Biol. 2012 May;46(5):614-22
6.Am J Respir Crit Care Med. 2014 Oct 15;190(8):848-50
7.Anal Biochem. 2006;355:71-80
8.Anticancer Res. 2013 Dec;33(12):5261-5271
9.Aust J Chem. 1983; 36:2465-2472
10. Bioorg Med Chem. 2010, 18, 46
11. Bioorg Med Chem. 2012, 20, 942
12. Bioorg Med Chem. 2014, 22, 2678
13. Bioorg Med Chem. 2015, 23, 290
14. Bioorg Med Chem Lett. 2014 Jan 15;24(2):565-70
15. Cardiovasc Ther. 2011 Apr;29(2):99-111
16. Dig Dis Sci. 2012 Oct;57(10):2580-91
17. Drug Discov Today. 2015 Nov;20(11):1382-90
18. Drug Metab Dispos. 2015 May;43(5):788-802
19. Equine Vet J. 2017 May;49(3):345-351
*20.* Exp Diabetes Res. 2012:758614
21. Expert Opin Ther Patents. 2010, vol. 20, pp. 941-956
22. FASEB J. 2015 Mar;29(3):1092-101
23. FASEB J.March 2008 22 (Meeting Abstract Supplement) 479.17
24. Inflamm Allergy Drug Targets. 2012 Apr;11(2):143-58
25. Int J Cardiol. 2012 Mar 8;155(2):181-7
26. J Agric Food Chem. 2011 Apr 13;59(7):2816-24
27. J Biol Chem. 2014 Dec 26;289(52):35826-38
28. J Cardiovasc Pharmacol. 2008 Oct;52(4):314-23
29. J Neurosci Res. 2017 Dec;95(12):2483-2492
30. J Pharmacol Exp Ther. 2016 Jun;357(3):529-36
31. J Pharmacol Exp Ther. 2017 Jun;361(3):408-416
32. J Surg Res. 2013 Jun 15;182(2):362-7
33. J Vet Pharmacol Ther. 2018 Apr;41(2):230-238
34. J Med Chem. 2012, vol. 55, pp. 1789-1808
35. J Neurosurg Anesthesiol. 2015 Jul; 27(3):222-240
36. Liebigs Ann Chem. 1973; 1839-1850
37. Liebigs Ann. 1995;523-535.
38. Life Sci. 2013 Jun 21;92(23):1145-50
39. Med Hypotheses, 2017 Oct;108:81-5
40. Mol Neurobiol. 2015 Aug;52(1):187-95
41. Mol Pharmacol. 2015 Aug;88(2):281-90
42. Nature. 2017 Dec 14;552(7684):248-252
43. Nutr Metab Cardiovasc Dis. 2012 Jul;22(7):598-604
44. Oncotarget. 2017 Sep 21;8(61):103236-60
45. Pharmacol Ther. 2017 Dec;180:62-76
46. Pharmacol Ther. 2017, vol 180, pp 62-76
47. Phytother Res. 2016 Jul;30(7):1119-27
48. PLoS One. 2013 Dec 11;8(12):e80922
49. PLoS One. 2014 May 13;9(5):e97529
50. Proc Natl Acad Sci U S A. 2005 Jul 12;102(28):9772-7
51. Proc Natl Acad Sci U S A. 2011 May 31;108(22):9038-43
52. Proc Natl Acad Sci U S A. 2015 Jul 21;112(29):9082-7
53. Proc Natl Acad Sci U S A. 2016 Mar 29;113(13):E1944-52
54. Proc Natl Acad Sci U S A. 2018 May 15;115(20):5283-5288
55. Prog Lipid Res. 2014 Jan;53:108-23
56. Prostaglandins Other Lipid Mediat. 2014 Oct;113-115:30-7
57. Prostaglandins Other Lipid Mediat. 2017 Jul;131:67-74
58. Prostaglandins Other Lipid Mediat. 2018 May;136:84-89
59. Recent Pat Cardiovasc Drug Discov. 2006 Jan;1(1):67-72.
60. Stroke. 2015 Jul;46(7):1916-22
61. Tetrahedron Lett. 1987, 28, 1585-1588.
62. Toxicol Appl Pharmacol. 2015 Jul 15;286(2):102-11
63. Toxicology. 2017 Aug 15;389:31-41

### Patent documents cited in the description

64. WO 00/23060 A2
65. WO 2007/009001 A1
66. WO 2003/002555 A1
67. WO 2017/120012 A1
68. WO 2016/133788 A1
69. DE 2210799
70. WO 2007/016496 A2

## Claims

1. A compound of formula (I) 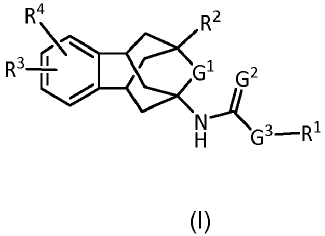 or a stereoisomer or a pharmaceutically acceptable salt thereof, wherein:
G¹ represents an oxygen atom or a methylene group or a single bond;
G² represents an oxygen atom or a sulphur atom;
G³ represents a radical selected from the group consisting of -NH-(CH₂)ₘ-, -O-(CH₂)ₘ- and -(CH₂)ₙ-;
m is an integer from 0 to 6;
n is an integer from 1 to 7
R¹ is a radical selected from the group consisting of:
a)C₆-C₁₀ aryl which may be optionally substituted by 1 to 4 substituents selected from the group consisting of halogen atoms, C₁-C₆ acyl, nitro (NO₂), cyano (C≡N), trifluoromethyl (CF₃), trifluoromethoxy (OCF₃), pentafluorosulfanyl (SF₅), sulfonyl (SO₃H), fluorosulfonyl (SO₂F), carboxylic group (COOH), amino (NH₂), mono-C₁-C₆ alkylamino, di-C₁-C₆ alkylamino, C₁-C₆ alkoxy, C₁-C₆ alkyl, C₁-C₆ alkoxycarbonylmethyl and methylaminocarbonylpyridyloxy;
b)heteroaryl having from 2 to 11 carbon atoms and 1, 2 or 3 heteroatoms selected from the group consisting of N, O and S and which may be optionally substituted by 1 to 4 substituents selected from the group consisting of halogen atoms, C₁-C₆ acyl, nitro (NO₂), cyano (C≡N), trifluoromethyl (CF₃), trifluoromethoxy (OCF₃), pentafluorosulfanyl (SF₅), sulfonyl (SO₃H), fluorosulfonyl (SO₂F), carboxylic group (COOH), amino (NH₂), mono-C₁-C₆ alkylamino, di-C₁-C₆ alkylamino, C₁-C₆ alkoxy, C₁-C₆ alkyl and C₁-C₆ alkoxycarbonylmethyl;
c)saturated or partially unsaturated, monocyclic or bicyclic heterocyclyl having from 5 to 11 carbon atoms and 1, 2 or 3 heteroatoms selected from the group consisting of N, O and S and which may be optionally substituted by 1 to 4 substituents selected from the group consisting of halogen atoms, C₁-C₆ acyl, C₃-C₆ cycloalkyl-C(=O), nitro (NO₂), cyano (C≡N), trifluoromethyl (CF₃), trifluoromethylcarbonyl (CF₃CO), pentafluorosulfanyl (SF₅), sulfonyl (SO₃H), carboxylic group (COOH), amino (NH₂), mono-C₁-C₆ alkylamino, di-C₁-C₆ alkylamino, C₁-C₆ alkoxy, C₁-C₆ alkyl, C₁-C₆ alkoxycarbonylmethyl, C₁-C₆ alkylsulfonyl, C₃-C₆ cycloalkylsulfonyl, benzyl, heteroarylmethyl, pyridincarbonyl, phenylcarbonyl, tetrahydropyrancarbonyl, C₆-C₁₀ arylsulfonyl which may be optionally substituted by 1 to 2 substituents selected from the group consisting of halogen atoms, nitro (NO₂), cyano (C≡N), trifluoromethyl (CF₃), trifluoromethoxy (OCF₃), pentafluorosulfanyl (SF₅), sulfonyl (SO₃H), carboxylic group (COOH), amino (NH₂), mono-C₁-C₆ alkylamino, di-C₁-C₆ alkylamino, C₁-C₆ alkoxy, C₁-C₆ alkyl, C₁-C₆ alkoxycarbonylmethyl and phenyl which may be optionally substituted by 1 to 4 substituents selected from the group consisting of halogen atoms, C₁-C₆ acyl, nitro (NO₂), cyano (C≡N), trifluoromethyl (CF₃), trifluoromethoxy (OCF₃), pentafluorosulfanyl (SF₅), sulfonyl (SO₃H), fluorosulfonyl (SO₂F), carboxylic group (COOH), amino (NH₂), mono-C₁-C₆ alkylamino, di-C₁-C₆ alkylamino, C₁-C₆ alkoxy, C₁-C₆ alkyl, C₃-C₆ cycloalkyl and C₁-C₆ alkoxycarbonylmethyl;
d)C₆-C₁₀ cycloalkyl which may be optionally substituted by 1 to 4 substituents selected from the group consisting of halogen atoms, C₁-C₆ acyl, nitro (NO₂), cyano (C≡N), trifluoromethyl (CF₃), trifluoromethoxy (OCF₃), pentafluorosulfanyl (SF₅), sulfonyl (SO₃H), carboxylic group (COOH), amino (NH₂), mono-C₁-C₆ alkylamino, di-C₁-C₆ alkylamino, C₁-C₆ alkoxy, C₁-C₆ alkyl, C₁-C₆ alkoxycarbonylmethyl, pyridinyloxy which may be unsubstituted or substituted by a group selected from COOH and CONHCH₃, and phenoxy which may be unsubstituted or substituted by COOH, COOR⁵, CONH₂, CN or OH;
R² is a radical selected from the group consisting of hydrogen or deuterium atoms, halogen atoms, methyl, hydroxy and C₁-C₆ alkoxy;
R³ and R⁴ are radicals which may be identical or different and which are independently selected from the group consisting of hydrogen atoms, halogen atoms, C₁-C₆ acyl, nitro (NO₂), cyano (C≡N), carboxylic group (COOH), hydroxy (OH), trifluoromethyl (CF₃), trifluoromethoxy (OCF₃), pentafluorosulfanyl (SF₅), sulfonyl (SO₃H), fluorosulfonyl (SO₂F), amino (NH₂), mono-C₁-C₆ alkylamino, di-C₁-C₆ alkylamino, C₁-C₆ alkoxy, C₁-C₆ alkyl and C₁-C₆ alkoxycarbonylmethyl;
or R³ and R⁴ may form together a radical -O-(CH₂)ₙ-O-, wherein n is an integer from 1 to 3;
R⁵ is a radical selected from C₁-C₆ alkyl and C₃-C₆ cycloalkyl.

2. A compound according to claim 1 wherein G¹ represents a methylene group.

3. A compound according to any one of claims 1 to 2 wherein G² represents an oxygen atom.

4. A compound according to any one of claims 1 to 3 wherein G³ represents a radical selected from the group consisting of -NH-(CH₂)ₘ- and -(CH₂)ₙ-, m is an integer from 0 to 6 and n is an integer from 1 to 7.

5. A compound according to claim 4 wherein G³ represents a radical-NH-(CH₂)ₘ- and m is an integer from 0 to 6.

6. A compound according to any one of claims 1 to 5 wherein, when G³ is selected from the group consisting of -NH-(CH₂)ₘ- and -O-(CH₂)ₘ-, m has a value of 0 and wherein G³ is -(CH₂)ₙ- n has a value of 1.

7. A compound according to any one of claims 1 to 6 wherein R¹ is selected from the group consisting of substituted or unsubstituted phenyl, substituted or unsubstituted cyclohexyl and substituted or unsubstituted piperidine.

8. A compound according to any one of claims 1 to 7 wherein R² is selected from the group consisting of hydrogen atoms, fluorine atoms, chlorine atoms, methyl, hydroxyl and C₁-C₃ alkoxy.

9. A compound according to any one of claims 1 to 8 wherein R³ and R⁴ are radicals which may be identical or different and which are independently selected from the group consisting of hydrogen atoms, halogen atoms, C₁-C₆ acyl, trifluoromethyl (CF₃), trifluoromethoxy (OCF₃), nitro (NO₂), amino (NH₂) and C₁-C₆ alkoxy.

10. A compound according to any one of claims 1 to 9 wherein R³ is hydrogen and R⁴ is a radical selected from the group consisting of hydrogen atoms, halogen atoms, C₁-C₆ acyl, trifluoromethyl (CF₃), trifluoromethoxy (OCF₃), nitro (NO₂), amino (NH₂) and C₁-C₆ alkoxy.

11. The compound according to any one of the claims 1 to 10, which is selected from the group consisting of:
i. *p*-tolyl (9-methyl-5,6,8,9,10,11-hexahydro-7*H*-5,9:7,11-dimethanobenzo[9]annulen-7-yl)carbamate
ii. 1-(9-methyl-5,6,8,9,10,11-hexahydro-7*H*-5,9:7,11-dimethanobenzo[9]annulen-7-yl)-3-(4-(trifluoromethyl)phenyl)thiourea
iii. 1-(1-acetylpiperidin-4-yl)-3-(5-methyl-1,5,6,7-tetrahydro-1,5:3,7-dimethanobenzo[e]oxonin-3(2*H*)-yl)urea
iv. 1-(1-acetylpiperidin-4-yl)-3-(1,5,6,7-tetrahydro-1,5:3,7-dimethanobenzo[*e*]oxonin-3(2*H*)-yl)urea
v. 1-(1-acetylpiperidin-4-yl)-3-(9-methyl-5,6,8,9,10,11-hexahydro-7*H*-5,9:7,11-dimethanobenzo[9]annulen-7-yl)urea
vi. 1-(1-acetylpiperidin-4-yl)-3-(9-hydroxy-5,6,8,9,10,11-hexahydro-7*H*-5,9:7,11-dimethanobenzo[9]annulen-7-yl)urea
vii. 1-(1-acetylpiperidin-4-yl)-3-(9-methoxy-5,6,8,9,10,11-hexahydro-7*H*-5,9:7,11-dimethanobenzo[9]annulen-7-yl)urea
viii. 1-(1-acetylpiperidin-4-yl)-3-(9-fluoro-5,6,8,9,10,11-hexahydro-7*H*-5,9:7,11-dimethanobenzo[9]annulen-7-yl)urea
ix. 1-(1-acetylpiperidin-4-yl)-3-(9-chloro-5,6,8,9,10,11-hexahydro-7*H*-5,9:7,11-dimethanobenzo[9]annulen-7-yl)urea
x. 4-(((1r,4r)-4-(3-(5-methyl-1,5,6,7-tetrahydro-1,5:3,7-dimethanobenzo[*e*]oxonin-3(2H)-yl)ureido)cyclohexyl)oxy)benzoic acid
xi. 4-(((1r,4r)-4-(3-(9-methyl-5,6,8,9,10,11-hexahydro-7*H*-5,9:7,11-dimethanobenzo[9]annulen-7-yl)ureido)cyclohexyl)oxy)benzoic acid
xii. 1-[1-(isopropylsulfonyl)piperidin-4-yl]-3-(9-methyl-5,6,8,9,10,11-hexahydro-7*H-*5,9:7,11-dimethanobenzo[9]annulen-7-yl)urea
xiii. 1-(1-benzylpiperidin-4-yl)-3-(9-methyl-5,6,8,9,10,11-hexahydro-7*H*-5,9:7,11-dimethanobenzo[9]annulen-7-yl)urea
xiv. 1-(2-acetyl-9-methyl-5,6,8,9,10,11-hexahydro-7*H*-5,9:7,11-dimethanobenzo[9]annulen-7-yl)-3-(1-acetylpiperidin-4-yl)urea
xv. 1-(1-acetylpiperidin-4-yl)-3-(9-methyl-2-nitro-5,6,8,9,10,11-hexahydro-7*H-*5,9:7,11-dimethanobenzo[9]annulen-7-yl)urea
xvi. 1-(1-acetylpiperidin-4-yl)-3-(2-amino-9-methyl-5,6,8,9,10,11-hexahydro-7*H-*5,9:7,11-dimethanobenzo[9]annulen-7-yl)urea
xvii. *tert*-butyl 4-(2-((9-methyl-5,6,8,9,10,11-hexahydro-7*H*-5,9:7,11-dimethanobenzo[9]annulen-7-yl)amino)-2-oxoethyl)piperidine-1-carboxylate
xviii. *N*-(9-methyl-5,6,8,9,10,11-hexahydro-7*H*-5,9:7,11-dimethanobenzo[9]annulen-7-yl)-2-(piperidin-4-yl)acetamide
xix. 2-[1-(isopropylsulfonyl)piperidin-4-yl]-*N*-(9-methyl-5,6,8,9,10,11-hexahydro-7*H-*5,9:7,11-dimethanobenzo[9]annulen-7-yl)acetamide
xx. 2-(1-acetylpiperidin-4-yl)-*N*-(9-methyl-5,6,8,9,10,11-hexahydro-7*H*-5,9:7,11-dimethanobenzo[9]annulen-7-yl)acetamide
xxi. 1-(9-methyl-6,7,8,9,10,11-hexahydro-5*H*-5,9:7,11-dimethanobenzo[9]annulen-7-yl)-3-(2,3,4-trifluorophenyl)urea
xxii. 1-(5-methyl-1,5,6,7-tetrahydro-1,5:3,7-dimethanobenzo[*e*]oxonin-3(2*H*)-yl)-3-(2,3,4-trifluorophenyl)urea
xxiii. 2-(1-benzylpiperidin-4-yl)-*N*-(9-methyl-5,6,8,9,10,11-hexahydro-7*H*-5,9:7,11-dimethanobenzo[9]annulen-7-yl)acetamide
xxiv. 1-(9-methyl-5,6,8,9,10,11-hexahydro-7*H*-5,9:7,11-dimethanobenzo[9]annulen-7-yl)-3-(1-propionylpiperidin-4-yl)urea
xxv. 1-(1-(4-acetylphenyl)piperidin-4-yl)-3-(9-methyl-5,6,8,9,10,11-hexahydro-7*H-*5,9:7,11-dimethanobenzo[9]annulen-7-yl)urea
xxvi. 1-(9-methyl-5,6,8,9,10,11-hexahydro-7*H*-5,9:7,11-dimethanobenzo[9]annulen-7-yl)-3-(1-(tetrahydro-2*H*-pyran-4-carbonyl)piperidin-4-yl)urea
xxvii. 1-(1-(2-fluorobenzoyl)piperidin-4-yl)-3-(9-methyl-5,6,8,9,10,11-hexahydro-7*H-*5,9:7,11-dimethanobenzo[9]annulen-7-yl)urea
xxviii. 1-((1R,3s,5S)-8-benzyl-8-azabicyclo[3.2.1]octan-3-yl)-3-(9-methyl-5,6,8,9,10,11-hexahydro-7*H*-5,9:7,11-dimethanobenzo[9]annulen-7-yl)urea
xxix. 1-(1-acetylpiperidin-4-yl)-3-(2-fluoro-9-methyl-5,6,8,9,10,11-hexahydro-7*H-*5,9:7,11-dimethanobenzo[9]annulen-7-yl)urea
xxx. 1-(1-acetylpiperidin-4-yl)-3-(2-methoxy-9-methyl-5,6,8,9,10,11-hexahydro-7*H-*5,9:7,11-dimethanobenzo[9]annulen-7-yl)urea
xxxi. 1-(1-acetylpiperidin-4-yl)-3-(1-fluoro-9-methyl-5,6,8,9,10,11-hexahydro-7*H-*5,9:7,11-dimethanobenzo[9]annulen-7-yl)urea
xxxii. 1-(1-acetylpiperidin-4-yl)-3-(2,3-dimethoxy-9-methyl-5,6,8,9,10,11-hexahydro-7*H-*5,9:7,11-dimethanobenzo[9]annulen-7-yl)urea
xxxiii. 1-(1-acetylpiperidin-4-yl)-3-(5,8,9,10-tetrahydro-5,8:7,10-dimethanobenzo[8]annulen-7(6*H*)-yl)urea
xxxiv. 1-(benzo[*d*]thiazol-2-yl)-3-(9-methoxy-5,6,8,9,10,11-hexahydro-7*H*-5,9:7,11-dimethanobenzo[9]annulen-7-yl)urea

12. A pharmaceutical or veterinary composition comprising a therapeutically effective amount of a compound as defined in any one of the claims 1 to 11.

13. A compound as defined in any one of the claims 1 to 11 or a composition according to claim 12 for use as a medicament.

14. A compound as defined in any one of the claims 1 to 11 or a composition according to claim 12 for use in the treatment or prevention in an animal, including a human, of a disease or disorder susceptible of improvement by inhibition of soluble epoxide hydrolase.

15. The compound for use according to claim 14, wherein the disease or disorder is selected from the group consisting of hypertension, atherosclerosis, pulmonary diseases, kidney diseases, stroke, pain, neuropathic pain, inflammation, pancreatitis in particular acute pancreatitis, immunological disorders, eye diseases in particular diabetic keratopathy, wet age-related macular degeneration and retinopathy such as premature retinopathy and diabetic retinopathy, cancer, obesity, including obesity-induced colonic inflammation, diabetes, metabolic syndrome, preeclampsia, anorexia nervosa, depression, erectile dysfunction, wound healing, NSAID-induced ulcers, emphysema, scrapie, Parkinson's disease, arthritis, arrhythmia, Alzheimer's disease, Raynaud's syndrome, cardiomyopathy, vascular cognitive impairment, mild cognitive impairment, inflammatory bowel diseases, cirrhosis, osteoporosis, chronic periodontitis, sepsis, seizure disorders (epilepsy), dementia, edema, attention-deficit hyperactivity disorder, schizophrenia, drug dependency, social anxiety, colitis, amyotrophic lateral sclerosis, chemotherapy induced side effects, laminitis, inflammatory joint pain and synovitis, endothelial dysfunction, subarachnoid hemorrhage, including aneurysmal subarachnoid hemorrhage, traumatic brain injury and diabetes-induced learning and memory impairment.
